(19) Europäisches Patentamt
European Patent Office
Office européen des brevets

(11) Veröffentlichungsnummer: **0 037 990**
**B1**

# (12) EUROPÄISCHE PATENTSCHRIFT

(45) Veröffentlichungstag der Patentschrift: **05.12.84**

(51) Int. Cl.³: **C 07 D 455/02**, A 61 K 31/445

(21) Anmeldenummer: **81102536.0**

(22) Anmeldetag: **03.04.81**

(54) **Phenyl-chinolizidine, entsprechende pharmazeutische Präparate, die Herstellung der Wirkstoffe sowie entsprechende Ausgangsverbindungen.**

(30) Priorität: **11.04.80 CH 2784/80**
**03.02.81 CH 701/81**

(43) Veröffentlichungstag der Anmeldung: **21.10.81 Patentblatt 81/42**

(45) Bekanntmachung des Hinweises auf die Patenterteilung: **05.12.84 Patentblatt 84/49**

(84) Benannte Vertragsstaaten: **AT BE CH DE FR GB IT LI LU NL SE**

(56) Entgegenhaltungen: **US - A - 3 692 791**

(73) Patentinhaber: **F. HOFFMANN-LA ROCHE & CO. Aktiengesellschaft, CH-4002 Basel (CH)**

(72) Erfinder: **Imhof, René, Dr., Bleumatthöhe 3, CH-5264 Gipf-Oberfrick (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 037 990 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue pharmakologisch aktive Phenyl-chinolizidine, ihre Herstellung, Zwischenprodukte bei der Herstellung der Phenyl-chinolizidine und Arzneimittel enthaltend solche Chinolizidine.

Die erfindungsgemässen Phenyl-chinolizidine sind Verbindungen der allgemeinen Formel

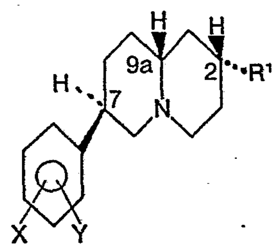

I

worin X, Y und $R^1$ folgendes bedeuten:

X Wasserstoff, Fluor, Chlor, nieder-Alkoxy, nieder-Alkyl oder Trifluormethyl,

Y Wasserstoff, Fluor, Chlor, nieder-Alkoxy oder nieder-Alkyl,

$R^1$ Acyl, Acylamino, das durch nieder-Alkyl oder gegebenenfalls durch Halogen, nieder-Alkyl oder nieder-Alkoxy substituiertes Phenyl am Stickstoffatom weiter substituiert sein kann, oder eine Gruppe A mit der Bedeutung

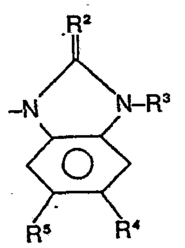

oder

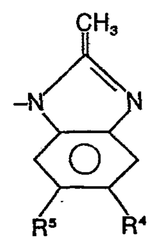

A

mit folgender Bedeutung für $R^2$–$R^5$:

$R^2$ Sauerstoff oder Schwefel,

$R^3$ Wasserstoff oder nieder-Alkyl,

$R^4$ und $R^5$ einer ist Wasserstoff, der andere ist Brom, Jod, Cyan, nieder-Alkoxycarbonyl oder Sulfamoyl,

in Form des Racemats oder der Enantiomeren, sowie Säureadditionssalze solcher Verbindungen.

Wie in der Formel I und in verschiedenen der nachfolgenden Formeln angegeben, befinden sich die Wasserstoffatome in den Stellungen 7 und 9a bzw. 7 und 2 transständig zueinander, wobei in den Formeln die 9a- und 2-Wasserstoffatome willkürlich in β-Stellung und das 7-Wasserstoffatom dementsprechend in α-Stellung angegeben sind. Die erfindungsgemässen Chinolizidine wie auch die entsprechenden Zwischenprodukte und Ausgangsstoffe sind jedoch nicht auf diese absolute Konfiguration beschränkt, sondern umfassen auch die entsprechenden enantiomeren Formen, also Verbindungen mit Wasserstoff in 9aα, 2α und 7β, sowie die Racemate dieser beiden enantiomeren Formen.

Unter nieder-Alkyl- bzw. nieder-Alkoxygruppen sind im vorliegenden Rahmen solche Gruppen zu verstehen, die 1–6, insbesondere 1–4 C-Atome enthalten, wobei die Gruppen mit 3 und mehr C-Atomen geradkettig oder verzweigt sein können. Der Acylrest $R^1$ kann sich von folgenden Carbonsäuren ableiten: von niederen Alkancarbonsäuren mit 1–6 C-Atomen, wie Essigsäure oder Propionsäure; von niederen Cycloalkancarbonsäuren mit 4–7 C-Atomen, wie Cyclopropancarbonsäure, Cyclohexancarbonsäure; von Benzoesäure, die durch Halogen, Amino, nieder-Alkoxy, Sulfamoyl und/oder nieder-Alkylsulfonyl substituiert sein kann, z.B. durch 4-Fluor, durch 2-Methoxy-4-amino-5-chlor, durch 2-Methoxy-5-sulfamoyl oder durch 2-Methoxy-5-äthylsulfonyl; und von Furan-2-carbonsäure, Thiophen-2-carbonsäure oder 2-Oxo-1-pyrrolidin-carbonsäure.

Die in Acylamino-Gruppen $R^1$ enthaltenen Acyl-Reste haben analoge Bedeutung wie oben angegeben für den Acylrest $R^1$, mit Ausnahme von ggf. substituiertem Benzoyl.

Als Säureadditionssalze der erfindungsgemässen Phenylchinolizidine kommen die pharmakologisch verträglichen Salze mit den üblicherweise zur Salzbildung verwendeten organischen und anorganischen Säuren in Betracht. Beispiele solcher Säuren sind: Mineralsäuren, wie Schwefelsäure, Salpetersäure, Phosphorsäure, Halogenwasserstoffsäuren (z.B. Salzsäure, Bromwasserstoffsäure); organische Säuren, wie Weinsäure, Citronensäure, aliphatische oder aromatische Sulfonsäuren, Maleinsäure, Mandelsäure usw. Die Salzbildung kann auf an sich bekannte Art erfolgen.

Untergruppen innerhalb der Formel I sind:

a) die Carboxamide der Formel

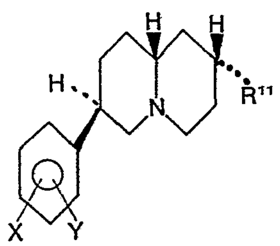

II

wobei X und Y dasselbe wie oben bedeuten und $R^{11}$ Acylamino darstellt,
in racemischer Form und in Form der Enantiomeren, sowie gegebenenfalls in Form von Säureadditionssalzen,

b) die Ketone der Formel

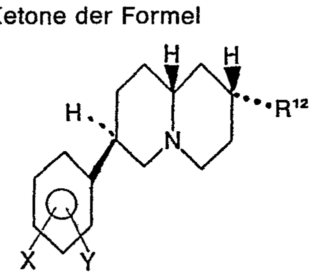

III

worin X und Y dasselbe wie oben bedeuten und $R^{12}$ Acyl darstellt,

in racemischer Form und in Form der Enantiomeren, sowie gegebenenfalls in Form von Säureadditionssalzen,

c) die Benzimidazolinon (und -thion)-Derivate der Formel

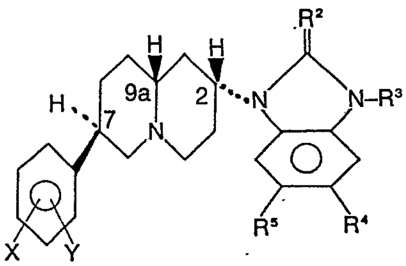

IV–1

worin X, Y sowie $R^2$–$R^5$ dasselbe wie oben bedeuten,
in racemischer Form und in Form der Enantiomeren, sowie gegebenenfalls in Form von Säureadditionssalzen,

d) die Methylbenzimidazol-Derivate der Formel

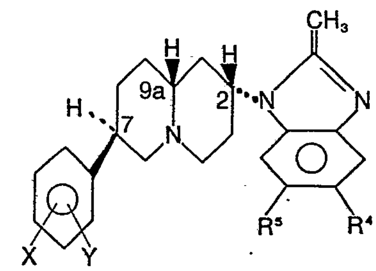

IV–2

mit obiger Bedeutung für X, Y, $R^4$ und $R^5$,
in racemischer Form und in Form der Enantiomeren, sowie gegebenenfalls in Form von Säureadditionssalzen.

Die Verbindungen der Formel I besitzen vielfältige pharmakologische Wirkung. So sind neuroleptische und/oder analgetische Wirkungen festgestellt worden. Besonders bevorzugt wegen ihrer pharmakologischen Wirkung sind Verbindungen der Formel I, worin X Wasserstoff und Y o-Chlor oder o-Fluor darstellen. Neuroleptische Wirkung liegt vor bei den Ketonen der Formel III, insbesondere worin $R^{12}$ p-Fluorbenzoyl darstellt. Neuroleptisch wirksam sind ebenfalls die Carboxamide der Formel II, insbesondere worin $R^{11}$ Cyclopropancarboxamido, 2-Thiophencarboxamido oder 2-Oxo-1-pyrrolidincarboxamido darstellt. Ebenfalls neuroleptisch wirksam sind die Benzimidazolinon (und -thion)-Derivate der Formel IV, insbesondere worin $R^4$ Brom, Jod oder Cyan und $R^5$ Wasserstoff darstellen, sowie auch die Methylbenzimidazol-Derivate der Formel IV-2. Analgetisch wirksam sind Carboxamide der allgemeinen Formel

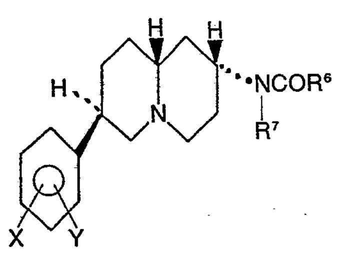

II–I

worin X und Y dasselbe wie oben bedeuten, $R^6$ niederes Alkyl, insbesondere Äthyl, und $R^7$ niederes Alkyl oder, insbesondere, Phenyl, das durch Halogen, insbesondere Chlor oder Fluor in 4-Stellung oder Chlor in 3-Stellung, niederes Alkyl oder niederes Alkoxy substituiert sein kann, darstellen.

Besonders bevorzugte Verbindungen sind:

Neuroleptisch aktive:

rac-1-[(9aβH)-7β-(o-Fluorphenyl)-octa-hydro-2H-chinolizin-2α-yl]-5-brom-2-benzimid-azolinon,
rac-1-[(9aβH)-7β-(o-Fluorphenyl)-octa-hydro-2H-chinolizin-2α-yl]-5-jod-2-benzimid-azolinon,
rac-1-[(9aβH)-7β-(o-Fluorphenyl)-octa-hydro-2H-chinolizin-2α-yl]-5-cyan-2-benzimid-azolinon,
rac-N-[(9aβH)-7β-(o-Chlorphenyl)-octa-hydro-2H-chinolizin-2α-yl]-cyclopropancarb-oxamid,
rac-N-[(9aβH)-7β-(o-Chlorphenyl)-octa-hydro-2H-chinolizin-2α-yl]-2-thiophencarb-oxamid,
rac-N-[(9aβH)-7β-(o-Chlorphenyl)-octa-hydro-2H-chinolizin-2α-yl]-2-oxo-1-pyrrolidincarb-oxamid,
rac-[(9aβH)-7β-(o-Chlorphenyl)-octa-hydro-2H-chinolizin-2α-yl]-p-fluorphenyl-keton.

Analgetisch aktive:

rac-N-[(9aβH)-7β-(o-Chlorphenyl)-octa-hydro-2H-chinolizin-2α-yl]-propionanilid,
rac-4′-Chlor-N-[(9aβH)-7β-(o-chlorphenyl)-octa-hydro-2H-chinolizin-2α-yl]-propion-anilid,
rac-3′-Chlor-N-[(9aβH)-7β-(o-chlorphenyl)-octa-hydro-2H-chinolizin-2α-yl]-propion-anilid,
rac-N-[(9aβH)-7β-(o-Fluorphenyl)-octa-hydro-2H-chinolizin-2α-yl]-propionanilid,
rac-4′-Fluor-N-[(9aβH)-7β-(o-fluorphenyl)-octahydro-2H-chinolizin-2α-yl]-propionanilid,
rac-4′-Fluor-N-[(9aβH)-7β-phenyl-octahydro-2H-chinolizin-2α-yl]-propionanilid,
rac-N-[(9aβH)-7β-(o-Fluorphenyl)-octa-hydro-2H-chinolizin-2α-yl]-acetanilid,
rac-N-[(9aβH)-7β-(o-Chlorphenyl)-octa-hydro-2H-chinolizin-2α-yl]-N-butylpropion-amid,
rac-N-[(9aβH)-7β-Phenyl-octahydro-2H-chinolizin-2α-yl]-propionanilid.

Die Phenyl-chinolizidine der Formel I werden erfindungsgemäss durch ein Verfahren hergestellt, welches dadurch gekennzeichnet ist, dass man

a) zur Herstellung von Carboxamiden der allgemeinen Formel

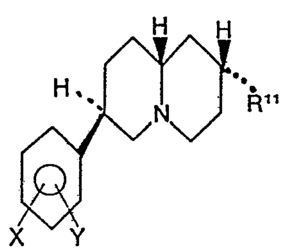

II

worin X und Y dasselbe wie oben bedeuten und $R^{11}$ Acylamino, das durch nieder-Alkyl oder gegebenenfalls durch Halogen, nieder-Alkyl oder nieder-Alkoxy substituiertes Phenyl am Stickstoffatom weiter substituiert sein kann, darstellt,
ein primäres oder sekundäres Amin der allgemeinen Formel

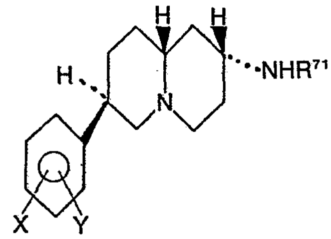

V

worin X und Y dasselbe wie oben bedeuten und $R^{71}$ Wasserstoff, nieder-Alkyl oder Phenyl, das durch Halogen, nieder-Alkyl oder nieder-Alkoxy substituiert sein kann, darstellt,
acyliert, oder dass man

b) zur Herstellung von Ketonen der allgemeinen Formel

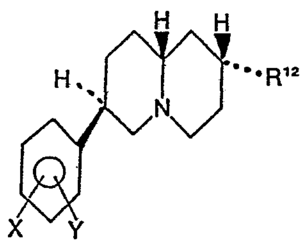

III

in der X und Y dasselbe wie oben bedeuten und $R^{12}$ Acyl darstellt,
eine Verbindung der allgemeinen Formel

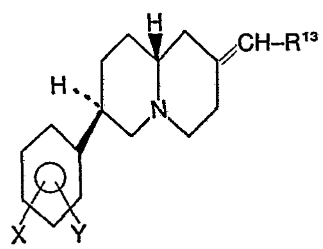

VI

in der X und Y dasselbe wie oben bedeuten und die Ylidengruppe $=CH-R^{13}$ dem Acylrest $R^{12}$, worin die Oxofunktion durch Wasserstoff ausgetauscht wurde, entspricht,
mit Diboran und Wasserstoffperoxid behandelt und das erhaltene Carbinol einer Oxidation unterwirft; oder dass man

c) zur Herstellung einer Verbindung der allgemeinen Formel

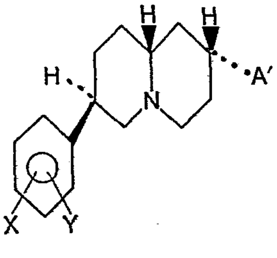

IV–3

worin X und Y dasselbe wie oben bedeuten und A′ wie A ist, wobei jedoch $R^2$ Sauerstoff darstellt und $R^4$ und $R^5$ von Brom und Jod verschieden sind,
eine Verbindung der allgemeinen Formel

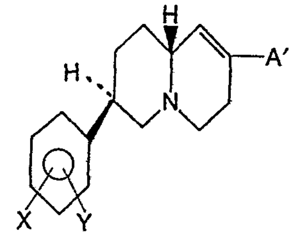

VII

worin X, Y und A′ dasselbe wie oben bedeuten,
katalytisch hydriert, oder dass man

d) zur Herstellung einer Verbindung der allgemeinen Formel

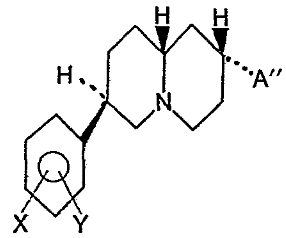

IV–4

worin X und Y dasselbe wie oben bedeuten und A″ wie A ist, wobei jedoch $R^2$ Sauerstoff und $R^3$ Wasserstoff darstellt,
ein Phenylen-diamin der allgemeinen Formel

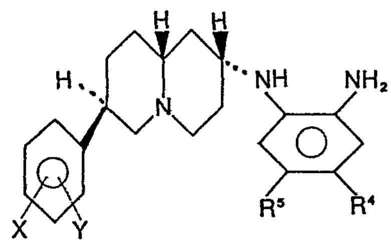

VIII

worin X, Y, $R^4$ und $R^5$ dasselbe wie oben bedeuten,
mit einem Cyclisierungsmittel der allgemeinen Formel

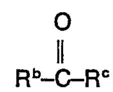

IX

worin $R^b$ und $R^c$ Halogen, Amino oder 1-Imidazolyl bedeuten,
oder mit Essigsäure oder einem Orthoessigsäureester kondensiert, oder dass man

e) zur Herstellung von Verbindungen der allgemeinen Formel

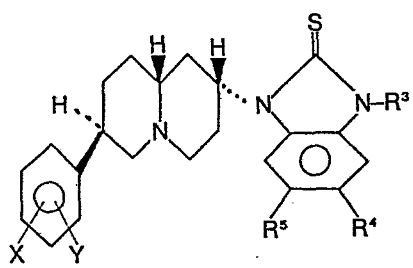

IV–5

worin X, Y sowie $R^3$–$R^5$ dasselbe wie oben bedeuten,

entweder eine entsprechende Oxo-Verbindung der allgemeinen Formel

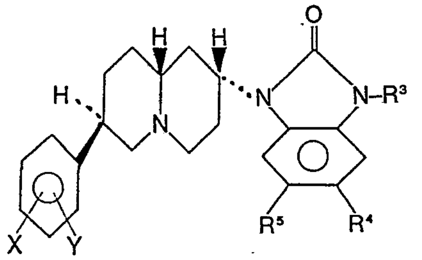

IV–6

worin X, Y sowie $R^3$–$R^5$ dasselbe wie oben bedeuten,

mit $P_2S_5$ umsetzt, oder ein Phenylen-diamin der obigen allgemeinen Formel VIII mit Thiophosgen, Thiocarbonyldiimidazol oder $CS_2$ umsetzt und anschliessend gegebenenfalls N-alkyliert, oder dass man

f) zur Herstellung von Verbindungen der allgemeinen Formel

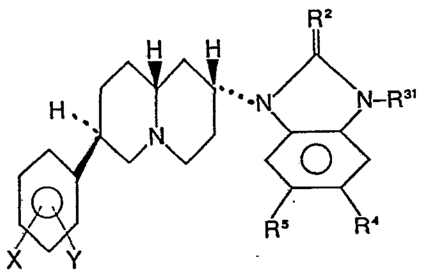

IV–7

worin X, Y, $R^2$, $R^4$ und $R^5$ dasselbe wie oben bedeuten und $R^{31}$ nieder-Alkyl darstellt,

eine Verbindung der allgemeinen Formel

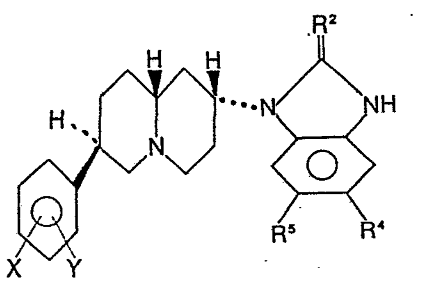

IV–8

worin X, Y, $R^2$, $R^4$ und $R^5$ dasselbe wie oben bedeuten,

N-alkyliert, wonach man erhaltene Racemate gegebenenfalls spaltet und erhaltene Basen gegebenenfalls in Säureadditionssalze überführt.

Sämtliche der in den vorstehenden Verfahrensvarianten a)–f) genannten Reaktionen können nach an sich bekannten Methoden durchgeführt werden.

Zur Acylierung eines primären oder sekundären Amins V gemäss der Verfahrensvariante a) kommen die üblicherweise für Acylierungsreaktionen verwendeten Agenzien in Betracht, beispielsweise ein reaktionsfähiges Derivat der entsprechenden Carbonsäure, z.B. das entsprechende Säurehalogenid, insbesondere das Säurechlorid, Säureanhydrid (insbesondere ein gemischtes Anhydrid, z.B. mit Chlorameisensäureäthylester) oder der entsprechende Säureester, z.B. der Methyl- oder Äthylester. Die Umsetzung erfolgt vorteilhaft in Gegenwart eines säurebindenden Mittels wie z.B. Triäthylamin, Pyridin oder Alkalimetallcarbonat. Es kann ebenfalls mit der entsprechenden Carbonsäure in Gegenwart eines Dehydratisierungsmittels, wie Dicyclohexylcarbodiimid, acyliert werden. Als Lösungsmittel für die Acylierung kommen die üblichen inerten Lösungsmittel in Betracht, z.B. Benzol, Toluol oder Diäthyläther. Die Reaktionstemperatur ist nicht kritisch und liegt vorzugsweise im Bereich zwischen etwa 0 °C und dem Siedepunkt des Reaktionsgemisches.

Zur Herstellung von Ketonen der allgemeinen Formel III wird eine Ylidenverbindung der allgemeinen Formel VI zuerst mit Diboran behandelt, wonach man das Additionsprodukt mit Wasserstoffperoxid zum entsprechenden carbinolischen Epimerengemisch oxidativ aufarbeitet und letzteres zum Keton oxidiert. Das Diboran erzeugt man bevorzugt in situ in bekannter Weise, beispielsweise aus einem Alkalimetallborhydrid und Bortrifluoridätherat in einem ätherischen Lösungsmittel, wie Äthylenglykoldimethyläther, bei einer Temperatur im Bereiche von etwa −20 °C und +50 °C. Für die Oxidation des Carbinols zum Keton verwendet man übliche anorganische oder organische Oxidationsmittel, z.B. Chromtrioxid, Mangandioxid, Alkalimetallpermanganat, Silbercarbonat oder Dimethylsulfoxid-Acetanhydrid-Triäthylamin. Das Keton der Formel V wird als Epimerengemisch (2αH+2βH) erhalten. Dieses Gemisch kann gewünschtenfalls in das stabilere 2βH-Epimere durch Behandeln mit einem Alkalimetallalkoholat umgewandelt werden. Die Oxidation wird in einem inerten Lösungsmittel durchgeführt, z.B. in Aceton; die anschliessende Epimerisierung erfolgt vorzugsweise in einem alkoholischen Lösungsmittel, z.B. in einem niederen Alkanol. Die Reaktionstemperatur für die Oxidation bzw. die Epimerisierung liegt bevorzugt im Bereich zwischen etwa 0 °C und dem Siedepunkt des Reaktionsgemisches.

Zur Herstellung der Benzimidazolderivate IV-3 nach der Verfahrensvariante c) kann man die $\Delta^1$-Doppelbindung einer Verbindung VII katalytisch hydrieren, wobei als Katalysatoren übliche Hydrierungskatalysatoren, wie $PtO_2$ oder Pd/C (z.B. 10%ig) verwendet werden können.

Zur Herstellung der Benzimidazolderivate IV-4 nach der Verfahrensvariante d) kann man ein Phenylendiamin VIII mit einem Cyclisierungsmittel IX, wie z.B. Phosgen, Carbonyldiimidazol oder Harnstoff, kondensieren, wobei ein Benzimidazolinon-Derivat des Typus IV-1 entsteht, oder auch mit Essigsäure oder einem Orthoessigsäureester, z.B. dem Trimethyl- oder Triäthylester, wobei ein Methylbenzimidazol-Derivat des Typus IV-2 erhalten wird.

Zur Herstellung des Thione IV-5 kann man gemäss Variante e) nach an sich bekannten Methoden den Sauerstoff des Imidazolrings einer Oxo-Verbindung IV-6 mit $P_2S_5$ gegen Schwefel austauschen, oder auf an sich bekannte Art ein Phenylendiamin VIII mit Thiophosgen, Thiocarbonyldiimidazol oder $CS_2$ umsetzen. Die anschliessende, fakultative N-Alkylierung an $R^3$ erfolgt wie nachstehend für die Verfahrensvariante f) angegeben.

Die Verfahrensvarianten d) und e) eignen sich besonders gut zur Herstellung von Verbindungen IV-4 bzw. IV-5, worin $R^4$ und $R^5$ verschieden ist.

Zur Herstellung von im Imidazolring N-alkylierten Verbindungen IV-7 nach der Verfahrensvariante f) kann man ein übliches N-Alkylierungsmittel, wie ein nieder-Alkylhalogenid, z.B. Methyljodid, verwenden.

Erhaltene Racemate lassen sich nach an sich bekannten Methoden in die optischen Antipoden spalten. Erhaltene Basen sind, ebenfalls nach herkömmlichen Methoden, in ihre entsprechenden Säureadditionssalze überführbar.

Die im folgenden als Zwischenprodukte bzw. Ausgangsstoffe genannten Verbindungen können, soweit sie nicht bekannt sind, nach an sich bekannten Methoden hergestellt werden. So können z.B. die in der Verfahrensvariante a) als Ausgangsmaterial verwendeten primären Amine V auf an sich bekannte Art durch Oximierung eines Ketons der allgemeinen Formel

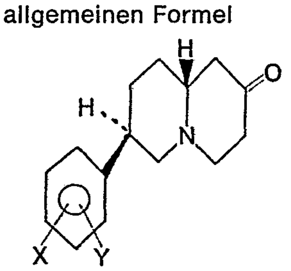

X

worin X und Y dasselbe wie oben bedeuten,
mit Hydroxylamin-hydrochlorid und Reduktion des erhaltenen Oxims zum Amin durch Hydrierung mit einem Edelmetallkatalysator, wie Raney-Nickel, erhalten werden. Die sekundäre Amine V werden beispielsweise in der Weise erhalten, dass man das Keton X einer reduktiven Aminierung mit einem primären Amin unterwirft. Als primäres Amin kommt vor allem Anilin und niedere Alkylamine, wie Methyl- oder Äthylamin, in Frage. Als Reduktionsmittel verwendet man vornehmlich ein Alkalimetallcyanborhydrid, z.B. Natriumcyanborhydrid. Das bevorzugte 2βH-Epimere kann aus einem erhaltenen Diastereomerengemisch aus Amins V chromatographisch getrennt werden oder auch durch fraktionierte Kristallisation entsprechender Additionssalze (z.B. der Hydrochloride).

Das Keton X seinerseits kann durch Decarboxylierung von β-Keto-estern der allgemeinen Formel XI mit der in dieser Formel angegebenen relativen Konfiguration bezüglich der Positionen 1, 7 und 9a

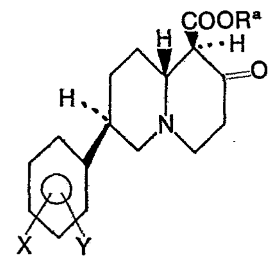

XI

worin X und Y dasselbe wie oben bedeuten und $R^a$ nieder-Alkyl darstellt,
erhalten werden (vgl. z.B. Beispiel 1e).

Die β-Keto-ester XI können ihrerseits aus den entsprechenden ungesättigten β-Keto-estern der allgemeinen Formel

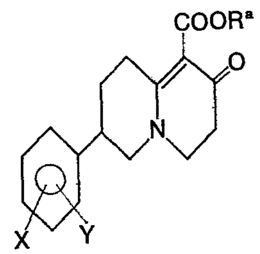

XII

worin X, Y und $R^a$ dasselbe wie oben bedeuten, auf an sich bekannte Art durch Hydrierung erhalten werden (vgl. z.B. das Beispiel 1f).

Die Ester XII können aus entsprechenden im Phenyl gegebenenfalls substituierten 5-Phenyl-2-piperidonen nach an sich bekannten Methoden (vgl. z.B. das Beispiel 1g) erhalten werden.

Die in der Verfahrensvariante b) als Ausgangsmaterial genannten Ylidenverbindungen VI erhält man aus den Ketonen X via einer Wittig-Reaktion mit einer Verbindung $R^{13}CH_2P(Phenyl)_3{}^+Cl^-$ und n-Butyllithium, beispielsweise nach den Angaben in Beispiel 7.

Das in der Verfahrensvariante d) verwendete Phenylendiamin VIII erhält man durch Umsetzung eines primären Amins V mit einem entsprechend substituierten o-Halogen-nitrobenzol, vorzugsweise einem o-Chlor- oder o-Fluor-nitrobenzolderivat und Reduktion der Nitrogruppe zu Amino, beispielsweise mit Salzsäure und Eisenpulver.

Die in der Verfahrensvariante f) als Ausgangsmaterial genannten Benzimidazolinone IV-8 können aus den entsprechenden ungesättigten Verbindungen der allgemeinen Formel XIII (mit der in dieser Formel angegebenen relativen Konfiguration in den Stellungen 7 und 9a)

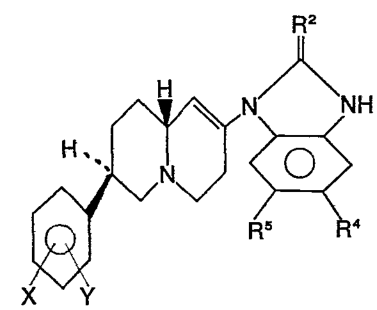

XIII

worin X, Y, $R^2$, $R^4$ und $R^5$ dasselbe wie oben bedeuten,
durch Hydrierung erhalten werden.

Durch N-Alkylierung von XIII wie in Verfahrensvariante f) erhält man N-alkylierte Ausgangsverbindungen VII (zur Verwendung in der Verfahrensvariante c).

Die Verbindungen XIII können ihrerseits aus den bereits genannten β-Keto-estern XI durch Erwärmen mit einem Diamin der allgemeinen Formel

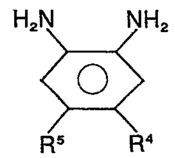

XIV

worin $R^4$ und $R^5$ dasselbe wie oben bedeuten, erhalten werden.

Die neuen Zwischenprodukte bzw. Ausgangsstoffe der Formeln V, VI, VII und VIII bilden ebenfalls Gegenstand der vorliegenden Erfindung.

Die erfindungsgemässen Phenyl-chinolizidine der Formel I zeichnen sich durch wertvolle pharmakologische Eigenschaften aus. Sie sind neuroleptisch, zentraldämpfend tranquilisierend, antiemetisch und/oder analgetisch wirksam und können demgemäss als Antipsychotica bzw. Antiemetica bzw. Analgetica bei der Bekämpfung von Psychosen und Neurosen bzw. von Übelkeit bzw. von Schmerz Verwendung finden.

Die neuroleptischen Eigenschaften der Verbindungen wurden zahlenmässig mit den nachstehend beschriebenen Tests ermittelt:

«Pole Climbing» Test (Ratte)

Zur Versuchsdurchführung wurde pro Dosis eine Gruppe von 10 Ratten eingesetzt, die daraufhin trainiert worden waren, durch Hinausspringen an eine vertikale isolierte Stange im Versuchskäfig, dem unmittelbar nach einem akustischen Signal (konditionierter Reiz), via Bodengitter ausgelösten Elektroschock (unkonditionierter Reiz) zu entkommen. Die Hemmung der konditionierten Reaktion bei 50% der Tiere während einer Beobachtungszeit von 6 Stunden wird durch den Parameter ED 50 BCR, die Hemmung der unkonditionierten Reaktion durch den Parameter ED 10 BUR bestimmt.

Spiroperidol-binding Test (in vitro)

In den in vitro Versuchen wurde Kalbsstriatum also Rezeptor verwendet (Lit.: Creese et al., Life Sci. 17, 993 (1975) und analog der von Fields et al., Brain Research 136, 578 (1977) beschriebenen Methoden mit [$^3$H]-Spiroperidol inkubiert. Mit einem Computerprogramm wurde aus 4 verschiedenen Konzentrationen in dreifacher Ausführung die IC 50 bestimmt, welche die Konzentration der Testsubstanz angibt, bei welcher eine 50%ige Ablösung der spezifischen Bindung von [$^3$H]-Spiroperidol am Rezeptor eintritt.

In der nachstehenden Tabelle A sind einige Versuchsresultate zusammengestellt.

Tabelle A

| Verbindung | Pole-Climbing BCR | BUR | Spiroperidol-Binding |
|---|---|---|---|
| | $ED_{50}$ i.p. (mg/kg) | $ED_{10}$ i.p. (mg/kg) | $IC_{50}$ (nanomolar) |
| rac-1-[(9aβH)-7β-(o-Fluorphenyl)-octahydro-2H-chinolizin-2α-yl]-5-brom-2-benzimidazolinon · HCl | 0,08 | 0,1 | 3,8 |
| rac-1-[(9aβH)-7β-(o-Fluorphenyl)-octahydro-2H-chinolizin-2α-yl]-5-jod-2-benzimidazolinon · HCl | 0,1 | 0,055 | 8,6 |
| rac-1-[(9aβH)-7β-(o-Fluorphenyl)-octahydro-2H-chinolizin-2α-yl]-5-cyan-2-benzimidazolinon · HCl | 0,65 | 1,0 | 50 |
| rac-N-[(9aβH)-7β-(o-Chlorphenyl)-octahydro-2H-chinolizin-2α-yl]-cyclopropancarboxamid | 6,0 | 5,5 | 550 |
| rac-N-[(9aβH)-7β-(o-Chlorphenyl)-octahydro-2H-chinolizin-2α-yl]-2-thiophencarboxamid | 18,0 | 25,0 | 123 |
| rac-N-[(9aβH)-7β-(o-Chlorphenyl)-octahydro-2H-chinolizin-2α-yl]-2-oxo-1-pyrrolidincarboxamid | 23,0 | $>30$ | |
| rac-[(9aβH)-7β-(o-Chlorphenyl)-octahydro-2H-chinolizin-2α-yl]-p-fluorphenylketon | 3,0 | 3,0 | 11 |

Die analgetischen Eigenschaften der Verbindungen wurden zahlenmässig mit den nachstehenden beschriebenen Tests ermittelt:

Writhing Test (Maus)

Zur Durchführung der Versuche wurden 8 männliche Mäuse (20–22 g) pro Dosis eingesetzt. 60 Minuten nach erfolgter oraler Verabreichung der Testsubstanz wurde den Tieren 10 ml/kg der Testlösung intraperitoneal injiziert. Anschliessend an eine Latenzzeit von 5 Minuten wurde die Zahl der Tiere registriert, bei denen während 5 Minuten nicht mehr als 1 charakteristisches Writhing-Symptom (konvulsive Streckbewegung des Körpers) auftrat. Die ED 50 gibt diejenige Dosis an, bei welcher 50% der Tiere nicht mehr als 1 Writhing zeigen.

In der nachstehenden Tabelle B sind einige Versuchsresultate zusammengestellt:

Tabelle B

| Verbindung | Writhing 60′ $ED_{50}$ p.o. (mg/kg) |
|---|---|
| rac-N-[(9aβH)-7β-(o-Chlorphenyl)-octahydro-2H-chinolizin-2α-yl]-propionanilid | 0,12 |
| rac-4′-Chlor-N-[(9aβH)-7β-(o-chlorphenyl)-octahydro-2H-chinolizin-2α-yl]-propionanilid | 1,3 |
| rac-3′-Chlor-N-[(9aβH)-7β-(o-chlorphenyl)-octahydro-2H-chinolizin-2α-yl]-propionanilid | 0,95 |
| rac-N-[(9aβH)-7β-(o-Fluorphenyl)-octahydro-2H-chinolizin-2α-yl]-propionanilid | 0,08 |
| rac-4′-Fluor-N-[(9aβH)-7β-(o-fluorphenyl)-octahydro-2H-chinolizin-2α-yl]-propionanilid | 0,17 |
| rac-4′-Fluor-N-[(9aβH)-7β-phenyloctahydro-2H-chinolizin-2α-yl]-propionanilid | 0,006 |

Die erfindungsgemässen Verbindungen der Formel I in Form des Racemats oder der Enantiomeren, sowie in Form der freien Basen wie von Säureadditionssalzen können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, lokal oder perkutan, z.B. in Form von Salben, Crèmes, Gelées, Lösungen, oder parenteral, z.B. in Form von Injektionslösungen, erfolgen.

Zur Herstellung von Tabletten, Lacktabletten, Dragées und Hartgelatinekapseln können die erfindungsgemässen Verbindungen mit pharmazeutisch inerten, anorganischen oder organischen Excipientien verabreicht werden. Als solche Excipientien kann man z.B. für Tabletten, Dragées und Hartgelatinekapseln Lactose, Maisstärke oder Derivate davon, Talk, Stearinsäure oder deren Salze usw. verwenden. Für Weichgelatinekapseln eignen sich als Excipientien z.B. vegetabile Öle, Wachse, Fette, halbfeste und flüssige Polyole usw.; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker, Glukose und dergleichen. Für Injektionslösungen eignen sich als Excipientien z.B. Wasser, Alkohole, Polyole, Glycerin, vegetabile Öle usw. Für Suppositorien, lokale oder perkutane Anwendungsformen eignen sich als Excipientien z.B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

In den genannten pharmazeutischen Präparaten kann die Dosierung etwa im Bereich von 0,5–100 mg liegen. Bei oraler Applikation kommen Wirkstoffmengen von etwa 0,05 mg/kg bis etwa 10 mg/kg pro Tag und bei parenteraler Applikation solche von etwa 0,01 mg/kg bis 1 mg/kg pro Tag in Betracht.

Beispiel 1

a) Eine Lösung von 4,80 g
rac-(9aβH)-7β-(o-Fluorphenyl)octahydro-2α-(2-amino-4-jod-anilino)-2H-chinolizin
in 80 ml Methylenchlorid wird mit 3,0 g N,N′-Carbonyl-diimidazol (93%ig) versetzt und über Nacht bei Raumtemperatur gerührt. Das ausgefallene Reaktionsprodukt wird nach 20 Stunden abfiltriert, mit 3 × 100 ml Methylenchlorid gewaschen und anschliessend getrocknet. Man erhält kristallines
rac-1-[(9aβH)-7β-(o-Fluorphenyl)-octa-hydro-2H-chinolizin-2α-yl-5-jod-2-benz-imidazolinon.
Smp. 271–274 °C. Das Hydrochlorid schmilzt bei 265–267 °C.

Wenn anstelle von
rac-(9aβH)-7β-(o-Fluorphenyl)octahydro-2α-(2-amino-4-jod-anilino)-2H-chinolizin
entsprechend substituierte Phenylendiamine mit N,N′-Carbonyl-diimidazol umgesetzt werden, gelangt man zu folgenden Produkten:
rac-1-[(9aβH)-7β-($R_a$)octahydro-2H-chinolizin-2α-yl]-(B)-2-benzimidazolinon:

| | | Smp., °C |
|---|---|---|
| $R_a$ = o-Fluorphenyl | B = 5-brom | 296–299 (HCl-Salz) |
| o-Fluorphenyl | B = 5-Äthoxycarbonyl | 277–280 (HCl-Salz) |
| o-Fluorphenyl | B = 5-Cyan | >300 (HCl-Salz) |
| o-Fluorphenyl | B = 5-Sulfamoyl | 290–295 (Base) |

b) Das als Ausgangsmaterial in Beispiel 1a verwendete
rac-(9aβH)-7β-(o-Fluorphenyl)octahydro-2α-(2-amino-4-jod-anilino)-2H-chinolizin
kann wie folgt erhalten werden:

5,1 g rac-(9aβH)-7β-(o-Fluorphenyl)octahydro-2α-(2-nitro-4-jod-anilino)-2H-chinolizin
werden in 100 ml Methanol, 2,0 ml konz. Salzsäure und 1,75 g Eisen-Pulver während 4 Stunden unter Rückflussbedingungen erhitzt. Anschliessend verteilt man das Reaktionsgemisch zwischen 0,5N Natronlauge und Chloroform. Die Chloroform-Extrakte werden über wasserfreiem Natriumcarbonat getrocknet und dann eingedampft. Aus Äthanol-n-Hexan kristallisiert das
rac-(9aβH)-7β-(o-Fluorphenyl)octahydro-2α-(2-amino-4-jod-anilino)-2H-chinolizin.
Smp. 106–110 °C.

Analog kann
rac-(9aβH)-7β-(o-Fluorphenyl)octahydro-2α-(2-amino-4-brom-anilino)-2H-chinolizin,
Smp. 153–155 °C, hergestellt werden, ebenso das entsprechende

| | |
|---|---|
| 4-Carbonitril | Smp.: 190–193 °C |
| 4-Sulfonsäureamid | Smp.: 226–228 °C |
| 4-Carbonsäureäthylester | Smp.: 149–156 °C |

c) Das als Ausgangsmaterial in Beispiel 1b eingesetzte
rac-(9aβH)-7β-(o-Fluorphenyl)octahydro-2α-(2-nitro-4-jod-anilino)-2H-chinolizin
kann wie folgt erhalten werden:
7,5 g rac-(9aβH)-7β-(o-Fluorphenyl)-2α-(amino)-octahydro-2H-chinolizin
werden in 75 ml Cyclohexanol (99%) gelöst, mit 6,40 g Natriumcarbonat (wasserfrei) versetzt und auf 160 °C erwärmt. Bei dieser Temperatur tropft man innerhalb von 3 Stunden eine Lösung von 9,2 g 2-Chlor-5-jod-nitrobenzol in 75 ml Cyclohexanol (>99%) zu. Anschliessend wird die Reaktionslösung für weitere 14 Stunden bei 160 °C gehalten. Nach Abkühlen auf Raumtemperatur wird vom anorganischen Rückstand abfiltriert und das Filtrat im Vakuum eingedampft. Den Abdampfrückstand löst man in 80 ml heissem Äthylacetat und 100 ml Methylenchlorid, setzt 2 g Aktivkohle zu und kocht 5 Minuten am Rückfluss. Danach wird heiss filtriert und das Filtrat auf 100 ml eingeengt. Es kristallisiert
rac-(9aβH)-7β-(o-Fluorphenyl)octahydro-2α-(2-nitro-4-brom-anilino)-2H-chinolizin.
Smp. 175–177 °C. Durch Chromatographie der Mutterlauge an 150 g Kieselgel mit n-Hexan/Diäthyläther 2 : 1 wird nochmals Produkt erhalten.

Falls anstelle von 2-Chlor-5-jod-nitrobenzol entsprechend substituierte o-Chlor-nitrobenzole eingesetzt werden, so erhält man folgende Produkte:

| | | Smp., °C |
|---|---|---|
| $R_a$ = o-Fluorphenyl | B = 2-nitro-4-brom-anilino | 175–117 |
| | 2-nitro-4-äthoxycarbonyl | 135–140 |
| | 2-nitro-4-cyan | 215–219 |
| | 2-nitro-4-sulfamoyl | 235–237 |

d) Das als Ausgangsmaterial in Beispiel 1c eingesetzte
rac-(9aβH)-7β-(o-Fluorphenyl)-2α-(amino)-octahydro-2H-chinolizin
kann wie folgt erhalten werden:
84,3 g rac-(9aβH)-7β-(o-Fluorphenyl)octahydro-2H-chinolizin-2-on
werden in 1700 ml Methanol mit 47,4 g Hydroxylamin-Hydrochlorid und 94,3 g Kaliumcarbonat (wasserfrei) während 1½ Stunden auf Rückfluss erhitzt. Anschliessend destilliert man das Methanol ab und verteilt den Rückstand zwischen 750 ml Wasser, 600 ml Chloroform und 150 ml Isopropanol. Die wässrige Phase wird zweimal mit je 250 ml eines Gemisches aus 200 ml Chloroform und 50 ml Isopropanol nachextrahiert, die gesammelten organischen Phasen über Magnesiumsulfat getrocknet und anschliessend eingedampft. Es resultieren 85,3 g Oxim, welches in 1,5 l Tetrahydrofuran, 0,5 l Äthanol sowie 1 l (5% g/g Ammoniak in Äthanol gelöst und mit 45 g Raney-Nickel während 30 Stunden hydriert wird (Raumtemperatur, Atmosphärendruck). Danach wird vom Katalysator abfiltriert und das Filtrat eingedampft. Aus dem Rückstand können durch Lösen in 400 ml Äthanol und Addieren von 130 ml 5N Chlorwasserstoff in Äthanol 53,5 g des Dihydrochlorid-Salzes von
rac-(9aβH)-7β-(o-Fluorpenyl)-2α-(amino)-octahydro-2H-chinolizin
auskristallisiert werden. Smp. 299–302 °C. Das diastereomere
rac-(9aβH)-7β-(o-Fluorphenyl)-2β-(amino)-octahydro-2H-chinolizin
ist als Dihydrochlorid in der Mutterlauge gelöst.

Wenn anstelle von
rac-(9aβH)-7β-(o-Fluorphenyl)octahydro-2H-chinolizin-2-on
entsprechend substituierte Chinolizidinone aminiert werden, so erhält man folgende Produkte:
rac-(9aβH)-7β-($R_a$)-2α-(amino)octahydro-2H-chinolizin:
$R_a$ = o-Chlorphenyl Smp. °C (· 2HCl): 293–296 °C

e) Das als Ausgangsmaterial in Beispiel 1d verwendete
rac-(9aβH)-7β-(o-Fluorphenyl)octa-hydro-2H-chinolizin-2-on
kann wie folgt erhalten werden:
76,2 g Methyl-rac-(1αH,9aβH)-7β-(o-Fluorphenyl)-octahydro-2-oxo-2H-chinolizin-1-carboxylat
werden in 1,2 l 4N Salzsäure gelöst und 7 Stunden am Rückfluss gekocht. Die abgekühlte Reaktionslösung wird anschliessend auf Eis gegossen und mit konz. Natronlauge alkalisch gestellt. Extraktion mit 3 × 500 ml Methylenchlorid

und Eindampfen der über Magnesiumsulfat getrockneten organischen Phase ergeben 68 g Rohprodukt. Aus Äther-Hexan kann
rac-(9aβH)-7β-(o-Fluorphenyl)octahydro-2H-chinolizin-2-on
kristallisiert werden. Smp. 74–76 °C.

Wenn anstelle von
Methyl-rac-(1αH,9aβH-7β-(o-chlorphenyl)-octahydro-2-oxo-2H-chinolizin-1-carboxylat
äquimolare Mengen entsprechend substituierte β-Ketoester decarboxyliert werden, erhält man folgende Produkte:

rac-(9aβH)-7β-($R_a$)octahydro-2H-chinolizin-2-on:

| | | Smp. °C |
|---|---|---|
| $R_a$ = | phenyl | 71– 72 |
| | p-chlorphenyl | 80– 82 |
| | p-trifluormethylphenyl | 103–104 |
| | o-methylphenyl IR(Film) | 1726 $cm^{-1}$ |
| | m-methoxyphenyl IR(Film) | 1726 $cm^{-1}$ |
| | 2,4-dichlorphenyl | 116–117 |
| | 2,6-dichlorphenyl | 116–118 |
| | o-chlorphenyl | 80– 82 |

f) Das im Beispiel 1e als Ausgangsmaterial verwendete Octahydro-carboxylat kann seinerseits aus dem entsprechenden Hexahydro-carboxylat wie folgt erhalten werden:
180 g Methyl-rac-7-(o-fluorphenyl)-3,4,6,7,8,9-hexahydro-2-oxo-2H-chinolizin-1-carboxylat
suspendiert man in 3,6 l Monoglym(Dimethoxyäthan), kühlt auf −30 °C und addiert unter Rühren 1,33 l DIBAH (Diisobutylaluminiumhydrid, 20%ige Lösung in Toluol). Anschliessend wird 1 Stunde bei −20 bis −30 °C gerührt und dann bei dieser Temperatur mit 1,72 l 2N Natronlauge hydrolysiert. Zur Aufarbeitung verteilt man zwischen 25 l Wasser und 20 l Chloroform; die organische Phase wird nochmals mit 2 × 5 l Wasser gewaschen, über Magnesiumsulfat getrocknet und eingedampft. Das Rohprodukt wird aus Äther-hexan kristallisiert. Durch Chromatographie der Mutterlauge an 1 kg Kieselgel mit Essigester wird nochmals Produkt erhalten. Man erhält
Methyl-rac-(1αH,9aβH)-7β-(o-fluorphenyl)-octahydro-2-oxo-2H-chinolizin-1-carboxylat,
Smp. 109–110 °C.

Wenn anstelle von
Methyl-rac-7-(o-fluorphenyl)-3,5,6,7,8,9-hexahydro-2-oxo-2H-chinolizin-1-carboxylat
äquimolare Mengen entsprechend substituierter Edukte mit DIBAH reduziert werden, gelangt man zu folgenden Verbindungen:
Methyl-rac-(1αH,9aβH)-7β-($R_a$)octahydro-2-oxo-2H-chinolizin-1-carboxylat:

| | | Smp. °C |
|---|---|---|
| $R_a$ = | phenyl | 114–116 |
| | p-chlorphenyl | 102–105 |
| | p-trifluormethylphenyl | 108–111 |
| | o-methylphenyl | 94– 98 |
| | m-methoxyphenyl IR(Film) | 1752, 1723, 1660 $cm^{-1}$ |
| | 2,4-dichlorphenyl | 116–118 |
| | 2,6-dichlorphenyl | 130–131 |
| | o-chlorphenyl | 122–124 |

g) Das im Beispiel 1f als Ausgangsmaterial verwendete Hexahydro-carboxylat kann seinerseits wie folgt erhalten werden:

150 g 5-(o-Fluorphenyl)-2-piperidon werden in 2 l Methylenchlorid gelöst und zu 410 g Triäthyloxoniumtetrafluorborat (Meerweinsalz) in 1 l Methylenchlorid bei Zimmertemperatur unter Rühren innerhalb von 60 Minuten zugetropft. Anschliessend kocht man 4 Stunden am Rückfluss und belässt weitere 15 Stunden bei Zimmertemperatur. In die auf 0 °C gekühlte Lösung werden sodann 372 g Kaliumcarbonat in 370 ml Wasser zugetropft, wobei intensiv gerührt wird. Man lässt weitere 2 Stunden bei Zimmertemperatur rühren, dekantiert anschliessend die Methylenchlorid-Phase und extrahiert den Rückstand mit 2 × 500 ml Methylenchlorid. Die über Kaliumcarbonat getrocknete Methylenchlorid-Phase wird zu einem öligen, teils kristallinen Rückstand eingeengt, sodann in 1 l Hexan aufgekocht und heiss filtriert. Aus dem Filtrat werden nach dem Abdampfen des Hexans das ölige Lactimäther erhalten: 3-(o-Fluorphenyl)-6-äthoxy-2,3,4,5-tetrahydropyridin.

Man löst den Lactimäther in 1,4 l Methanol, gibt 1,3 g wasserfreie p-Toluolsulfonsäure zu und addiert innerhalb von 60 Minuten 85 g 3-Oxo-5-pentensäure-methylester (Nazarov-Reagens). Nach 20 Stunden bei Raumtemperatur werden die leichtflüssigen Anteile im Vakuum entfernt, der Rückstand anschliessend in Methylenchlorid aufgenommen und mit 500 ml ges. Natriumcarbonat-Lösung gewaschen. Die über Magnesiumsulfat getrocknete Methylenchlorid-Phase wird wiederum eingeengt und der Rückstand aus Essigester-Äther = 4 : 1 (800 ml) kristallisiert. Man erhält
Methyl-rac-7-(o-fluorphenyl)-3,4,6,7,8,9-hexahydro-2-oxo-2H-chinolizin-1-carboxylat.
Smp. 165–167 °C.

Wenn anstelle von 5-(o-Fluorphenyl)-2-piperidon äquimolare Mengen entsprechend substituierter Piperidone verwendet wird, erhält man die folgenden Verbindungen:
Methyl-rac-7-($R_a$)-3,4,6,7,8,9-hexahydro-2-oxo-2H-chinolizin-1-carboxylat:

| | | Smp. °C |
|---|---|---|
| $R_a$ = | phenyl | 148–149 |
| | p-chlorphenyl | 185–187 |
| | p-trifluormethylphenyl | 144–145 |
| | o-methylphenyl | 183–184 |
| | m-methoxyphenyl | 133–134 |
| | 2,4-dichlorphenyl | 153–155 |
| | 2,6-dichlorphenyl | 159–162 |
| | o-chlorphenyl | 145–147 |

Beispiel 2

70 g rac-1-[(9aβ)-7β-(o-Fluorphenyl)octa-hydro-2H-chinolizin-2α-yl]-2-oxo-5-benzimidazolin-carbonsäureäthylester-Hydrochlorid
werden in 500 ml 5N Salzsäure während 96 Stunden unter Rückfluss gekocht. Anschliessend kühlt man auf 0 °C ab, saugt das Reaktionsprodukt über eine Nutsche ab, wäscht den Rückstand mit 3 × 100 ml Wasser und trocknet am Hochvakuum.

Es resultiert
rac-1-[(9aβH)-7β-(o-Fluorphenyl)octahydro-2H-chinolizin-2α-yl]-2-oxo-5-benzimidazoline-carbonsäure Hydrochlorid.
Smp. 285–295 °C.

Beispiel 3

4,10 g rac-(9aβH)-7β-(o-Chlorphenyl)-2α-(amino)-octahydro-2H-chinolizin
in 40 ml Chloroform gelöst werden bei 5 °C unter heftigem Rühren gleichzeitig mit 3,0 g 4-Fluorbenzoylchlorid in 30 ml Chloroform und 0,8 g Natriumhydroxid in 15 ml Wasser versetzt. Anschliessend rührt man während 45 Minuten bei 5 °C und 60 Minuten bei Zimmertemperatur weiter. Das Reaktionsgemisch wird anschliessend zwischen Chloroform und Wasser verteilt, die Chloroform-Phase über wasserfreiem Natriumcarbonat getrocknet und eingedampft. Durch Umkristallisation aus Äthylacetat erhält man kristallines
rac-N-[(9aβH)-7β-(o-Chlorphenyl)octahydro-2H-chinolizin-2α-yl]-p-fluorbenzamid.
Smp. 200–202 °C.
Für die vorstehend beschriebene Verbindung und ihre Herstellung wird kein Schutz begehrt.

Analog werden erhalten
rac-N-[(9aβH)-7β-(o-Chlorphenyl)octahydro-2H-chinolizin-2α-yl]cyclopropancarboxamid.
Smp. 193–195 °C.
rac-N-[(9aβH)-7β-(o-Chlorphenyl)octahydro-2H-chinolizin-2α-yl]-2-thiophencarboxamid.
Smp. 217–219 °C.

Beispiel 4

5,30 g rac-(9aβH)-7β-(o-Chlorphenyl)-2α-(amino)octahydro-2H-chinolizin in 100 ml Methylenchlorid werden tropfenweise zu 3,56 g N,N′-Carbonyldiimidazol in 100 ml Methylenchlorid zugetropft und anschliessend während 2 Stunden bei Zimmertemperatur weitergerührt. Dann giesst man das Reaktionsgemisch auf 1N Natronlauge und extrahiert zweimal mit je 300 ml Methylenchlorid. Die über wasserfreiem Natriumcarbonat getrocknete organische Phase wird anschliessend eingeengt. Als Zwischenprodukt erhält man rohes
rac-N-[(9aβH)-7β-(o-Chlorphenyl)octahydro-2H-chinolizin-2α-yl]imidazol-1-carboxamid.
Das rohe Zwischenprodukt behandelt man in 250 ml Toluol mit 12,7 g N-Trimethylsilyl-pyrrolidin-2-on bei 80 °C während 17 Stunden. Anschliessend wird eingedampft und das rohe Reaktionsgemisch über 1 kg Kieselgel Merck (0,063–0,2 mm) mit Chloroform-3% Methanol chromatographiert. Man erhält
rac-N-[(9aβH)-7β-(o-Chlorphenyl)octahydro-2H-chinolizin-2α-yl]-2-oxo-1-pyrrolidincarboxamid.
Smp. 184–186 °C.

Beispiel 5

4,20 g rac-N-[(9aβH)-7β-(o-Chlorphenyl)-octahydro-2H-chinolizin-2α-yl] anilin
in 100 ml abs. Toluol gelöst werden mit 3,4 ml Propionsäurechlorid und 3 ml Triäthylamin versetzt und während 6 Stunden auf 80 °C erwärmt. Anschliessend verteilt man die Reaktionslösung zwischen 2N Natronlauge und Chloroform und extrahiert die wässrige Phase nochmals mit Chloroform. Die über wasserfreiem Natriumcarbonat getrocknete organische Phase wird eingedampft und auf einer 100 g Kieselgelsäule mit Chloroform-1% Methanol chromatographiert. Man eluiert
rac-N-[(9aβH)-7β-(o-Chlorphenyl)octahydro-2H-chinolizin-2α-yl]propionanilid,
das sich aus Äther/n-Hexan kristallisieren lässt.
Smp. 133–135 °C.

Analog wird erhalten:
rac-4′-Chlor-N-[(9aβH)-7β-(o-chlorphenyl)-octahydro-2H-chinolizin-2α-yl]propionanilid.
Smp. (HBr-Salz): 286–271 °C.
rac-3′-Chlor-N-[(9aβH)-7β-(o-chlorphenyl)-octahydro-2H-chinolizin-2α-yl]-propionanilid.
Smp. (Basel) 124–126 °C.
rac-N-[(9aβH)-7β-(o-Fluorphenyl)-octahydro-2H-chinolizin-2α-yl]-propionanilid.
Smp. (HCl-Salz) 221–223 °C.
rac-4′-Fluor-N-[(9aβH)-7β-(o-fluorphenyl)-octahydro-2H-chinolizin-2α-yl]-propionanilid.
Smp. (HCl-Salz) 240–242 °C.
rac-4′-Fluor-N-[(9aβH)-7β-phenyl-octahydro-2H-chinolizin-2α-yl]-propionanilid.
Smp. (HCl-Salz) 238–240 °C.
rac-N-[(9aβH)-7β-(o-Fluorphenyl)-octahydro-2H-chinolizin-2α-yl]-acetanilid.
Smp. (HCl-Salz) 232–236 °C.
rac-N-[(9aβH)-7β-(o-Chlorphenyl)-octahydro-2H-chinolizin-2α-yl]-N-butylpropionamid.
Smp. (HCl-Salz) 175–177 °C.
rac-N-[(9aβH)-7β-Phenyl-octahydro-2H-chinolizin-2α-yl]-propionanilid.

Das im obigen Beispiel als Edukt eingesetzte Material kann wie folgt erhalten werden:

10,5 g rac-(9aβH)-7β-(o-Chlorphenyl)-octahydro-2H-chinolizin-2-on werden in 150 ml Methanol gelöst, mit 10 g Molekularsieb 3 Å und 5 mg Bromkresol «grün» versetzt. Anschliessend addiert man 4,10 g Anilin, setzt 1,77 g Natriumcyanborhydrid zu und lässt dann tropfenweise 2,2N HCl in Methanol (insgesamt 55 ml) zufliessen; als Mass für die Zutropfgeschwindigkeit wird der Bromkresol «grün»-Indikator verwendet, die auf den Umschlagsbereich grün-gelb eingestellt wird. Nach 72 Stunden Reaktionszeit wird zwischen 2N Natronlauge und Chloroform verteilt, die Chloroform-Extrakte über wasserfreiem Natriumcarbonat getrocknet und eingedampft. Das Rohprodukt chromatographiert man an 250 g Kieselgel, beginnend mit 1,5 l Äthylacetat-n-Hexan = 1 : 2; mit Äthylacetat-n-Hexan = 1 : 1 (1 lt) eluiert man das
rac-N-[(9aβH)-7β-(o-Chlorphenyl)-octahydro-2H-chinolizin-2α-yl]anilin.

Analog wird erhalten:
rac-4′-Chlor-N-[(9aβH)-7β-(o-chlorphenyl)-octahydro-2H-chinolizin-2α-yl]anilin.
rac-3′-Chlor-N-[(9aβH)-7β-(o-chlorphenyl)-octahydro-2H-chinolizin-2α-yl]anilin.
rac-N-[(9aβH)-7β-(o-Fluorphenyl)-octahydro-2H-chinolizin-2α-yl]anilin.
rac-4′-Fluor-N-[(9aβH)-7β-(o-fluorphenyl)-octahydro-2H-chinolizin-2α-yl]anilin.

rac-4'-Fluor-N-[(9aβH)-7β-phenyl-octahydro-2H-chinolizin-2α-yl]anilin.
rac-N-[(9aβH)-7β-(o-Fluorphenyl-octahydro-2H-chinolizin-2α-yl]anilin.
rac-N-[(9aβH)-7β-(o-Chlrophenyl)-octahydro-2H-chinolizin-2α-yl]anilin.
rac-N-[(9aβH)-7β-Phenyl-octahydro-2H-chinolizin-2α-yl]anilin.

Beispiel 6

14,5 g p-Fluorbenzyl-triphenyl-phosphoniumchlorid werden in 100 ml abs. Diäthyläther suspendiert und bei 0 °C mit 20 ml n-Butyllithium (1,8 molar) versetzt und 1 Stunde bei 0–5 °C gerührt. Anschliessend tropft man eine Lösung von 8,8 g

rac-(9aβH)-7β-(o-Chlorphenyl)octahydro-2H-chinolizin-2-on

in 150 ml abs. Diäthyläther zu und erwärmt dann während 4 Stunden auf Rückflusstemperatur. Die abgekühlte Lösung hydrolysiert man mit 30 ml gesättigter Ammoniumchlorid-Lösung und verteilt dann zwischen Äthylacetat und 0,5N Natronlauge. Die über wasserfreiem Magnesiumsulfat getrocknete Äthylacetat-Phase wird eingedampft und über 100 g Kieselgel mit Äthylacetat-n-Hexan = 1 : 1 chromatographiert. Man eluiert öliges E/Z-Gemisch von

rac-(9aβH)-7β-(o-Chlorphenyl)-2-[(p-fluorphenyl)-methylen]octahydro-2H-chinolizin.

Dieses Gemisch löst man in 115 ml Äthylenglycoldimethyläther (über Aluminiumoxid, Aktivitätsstufe I, neutral getrocknet), versetzt mit 1,77 g Natriumborhydrid und tropft bei Zimmertemperatur eine Lösung von 11,5 g Bortrifluorid-Ätherat in 77 ml Äthylenglycoldimethyläther zu. Nach 1 Stunde Rühren bei Zimmertemperatur kühlt man auf 0–5 °C ab und tropft vorsichtig eine Lösung von 1,23 g Kaliumhydroxid in 50 ml Wasser und anschliessend 4,6 ml Wasserstoffperoxid (60%) zu und erwärmt sodann auf Rückflusstemperatur. Das abgekühlte Reaktionsgemisch verteilt man zwischen 2N Natronlauge und Essigester, trocknet die organische Phase über wasserfreiem Magnesiumsulfat und dampft zur Trockene ein. Dieses Rohprodukt löst man in 240 ml Aceton, kühlt auf 0–5 °C und tropft 32 ml Jones-Reagens (hergestellt aus 80 g Chromtrioxid, 64 ml konz. Schwefelsäure, 300 ml Wasser) zu und rührt 1 Stunde bei 0–5 °C. Anschliessend verteilt man das Reaktionsgemisch zwischen 150 ml ges. Natriumacetat-Lösung und 250 ml Äthylacetat sowie 200 ml 2N Natronlauge. Die über wasserfreiem Magnesiumsulfat getrocknete organische Phase wird zur Trockene eingedampft. Das Rohprodukt wird nochmals in 30 ml Äthylalkohol gelöst und mit 0,3 g Natriumäthylat versetzt. Nach 3-stündigem Rühren bei Zimmertemperatur verteilt man zwischen 2N Natronlauge und Äthylacetat, trocknet die organische Phase über wasserfreiem Magnesiumsulfat, behandelt mit 1 g Aktivkohle unter Erwärmen auf 50 °C, filtriert ab und dampft zur Trockene ein. Durch Kristallisation aus Äthylalkohol-Äthylacetat erhält man

rac-(9aβH)-7β-(o-Chlorphenyl)octahydro-2H-chinolizin-2α-yl)p-fluorphenylketon

(Smp. 145–147 °C) erhalten. Aus der Mutterlauge kann durch Chromatographie an 100 g Kieselgel mit Äthylacetat eine weitere Fraktion Produkt erhalten werden. HCl-Salz (aus Methylenchlorid-Äthylacetat) Smp.: 256–258 °C.

Beispiel 7

| Tabletten | Pro Tablette |
|---|---|
| rac-(9aβH)-7β-(o-Chlorphenyl)octahydro-2H-chinolizin-2α-yl)p-fluorphenylketon · HCl | 100 mg |
| Lactose | 202 mg |
| Maisstärke | 80 mg |
| Hydrolysierte Maisstärke | 20 mg |
| Calciumstearat | 8 mg |
| Totalgewicht | 410 mg |

Der Wirkstoff, die Lactose, die Maisstärke und die hydrolysierte Maisstärke werden gemischt und mit Wasser zu einer zähen Paste granuliert. Diese Paste wird durch ein Sieb passiert und anschliessend bei 45 °C über Nacht getrocknet. Das getrocknete Granulat wird durch ein Sieb passiert und anschliessend mit dem Calciumstearat vermischt. Die erhaltene Mischung wird zu Tabletten von einem Gewicht von 410 mg und mit einem Durchmesser von etwa 10 mm gepresst.

Beispiel 8

| Tabletten | Pro Tablette |
|---|---|
| rac-1-[(9aβH)-7β-(o-Fluorphenyl)-octahydro-2H-chinolizin-2α-yl]-5-brom-2-benzimidazolinon · HCl | 10,0 mg |
| Lactose | 129,0 mg |
| Maisstärke | 50,0 mg |
| Gelatinierte Maisstärke | 8,0 mg |
| Calciumstearat | 3,0 mg |
| Totalgewicht | 200,0 mg |

Der Wirkstoff, die Lactose, die Maisstärke und die gelatinierte Maisstärke werden innig miteinander vermischt. Die Mischung wird durch eine Zerkleinerungsmaschine passiert und anschliessend mit Wasser zu einer dicken Paste befeuchtet. Die feuchte Masse wird durch ein Sieb passiert. Das feuchte Granulat wird bei 45 °C getrocknet. Das getrocknete Granulat wird mit dem Calciumstearat gründlich vermischt. Das Granulat wird nun zu Tabletten von einem Gewicht von 200 mg und einem Durchmesser von etwa 8 mm gepresst.

Beispiel 9

| Tabletten | pro Tablette |
|---|---|
| rac-1-[(9aβH)-7β-(o-Fluorphenyl)octahydro-2H-chinolizin-2α-yl]-5-jod-2-benzimidazolinon · HCl | 10,0 mg |
| Lactose | 129,0 mg |

| Tabletten | Pro Tablette |
|---|---|
| Maisstärke | 50,0 mg |
| Gelatinierte Maisstärke | 8,0 mg |
| Calciumstearat | 3,0 mg |
| Totalgewicht | 200,0 mg |

Der Wirkstoff, die Lactose, die Maisstärke und die gelatinierte Maisstärke werden innig miteinander vermischt. Die Mischung wird durch eine Zerkleinerungsmaschine passiert und anschliessend mit Wasser zu einer dicken Paste befeuchtet. Die feuchte Masse wird durch ein Sieb passiert. Das feuchte Granulat wird bei 45 °C getrocknet. Das getrocknete Granulat wird mit dem Calciumstearat gründlich vermischt. Das Granulat wird nun zu Tabletten von einem Gehalt von 200 mg und einem Durchmesser von etwa 8 mm gepresst.

Beispiel 10

| Tabletten | pro Tablette |
|---|---|
| rac-N-((9aβH)-7β-(o-Chlorphenyl)-octahydro-2H-chinolizin-2α-yl)propionanilid · HBr | 20,0 mg |
| Milchzucker | 115,0 mg |
| Maisstärke | 61,0 mg |
| Talk | 3,4 mg |
| Magnesiumstearat | 0,6 mg |
| Totalgewicht | 200,0 mg |

Die Ingredienzien werden innig miteinander vermischt und zu Tabletten von je 200 mg gepresst. Anschliessend werden sie mit Äthylcellulose und Carbowax überzogen.

Beispiel 11

| Kapseln | pro Kapsel |
|---|---|
| rac-1-[(9aβH)-7β-(o-Fluorphenyl)-octahydro-2H-chinolizin-2α-yl]-5-brom-2-benzimidazolinon · HCl | 10,0 mg |
| Lactose | 175,0 mg |
| Maisstärke | 30,0 mg |
| Talk | 5,0 mg |
| Totalgewicht | 220,0 mg |

Der Wirkstoff, die Lactose und die Maisstärke werden innig miteinander vermischt und durch eine Zerkleinerungsmaschine passiert. Die Mischung wird nun mit dem Talk gründlich vermischt und in harte Gelatinekapseln gefüllt.

Beispiel 12

| Kapseln | pro Kapsel |
|---|---|
| rac-N-((9aβH)-7β-(o-Chlorphenyl)octahydro-2H-chinolizin-2α-yl)propionanilid | 10,0 mg |
| Lactose | 175,0 mg |
| Maisstärke | 30,0 mg |
| Talk | 5,0 mg |
| Totalgewicht | 220,0 mg |

Der Wirkstoff, die Lactose und die Maisstärke werden innig miteinander vermischt und durch eine Zerkleinerungsmaschine passiert. Die Mischung wird nun mit dem Talk gründlich vermischt und in harte Gelatinekapseln gefüllt.

Beispiel 13

Jede 1 ml Ampulle enthält:

| | | |
|---|---|---|
| rac-1-[(9aβH)-7β-(o-Fluorphenyl)-octahydro-2H-chinolizin-2α-yl]-5-brom-2-benzimidazolinon · HCl | 0,204 mg (2% Überschuss) | |
| Glucose für Injektionszwecke | 40,0 | mg |
| Wasser für Injektionszwecke q.s. ad | 1,0 | mg |

2,04 g Wirkstoff werden mit 400 g Glucose versetzt, in Wasser für Injektionszwecke gelöst und mit Wasser für Injektionszwecke auf ein Volumen von 10 000 ml ergänzt. Die Lösung wird entweder steril filtriert, in farblose Ampullen abgefüllt, mit Stickstoff begast und versiegelt oder in farblose Ampullen abgefüllt, mit Stickstoff begast, versiegelt und anschliessend 30 Minuten mit strömendem Dampf sterilisiert oder bei 120 °C autoklaviert.

Beispiel 14

Parenterale Zubereitungsform (0,5 mg)

Jede 1 ml Ampulle enthält:

| | | |
|---|---|---|
| rac-N-[(9aβH)-7β-(o-Chlorphenyl)octahydro-2H-chinolizin-2α-yl]propionanilid | 0,510 mg (2% Überschuss) | |
| Glucose für Injektionszwecke | 40,0 | mg |
| Wasser für Injektionszwecke q.s. ad | 1,0 | ml |

5,10 g Wirkstoff werden mit 400 g Glucose versetzt, in Wasser für Injektionszwecke gelöst und mit Wasser für Injektionszwecke auf ein Volumen von 10 000 ml ergänzt. Die Lösung wird entweder steril filtriert, in farblose Ampullen abgefüllt, mit Stickstoff begast und versiegelt oder in farblose Ampullen abgefüllt, mit Stickstoff begast, versiegelt und anschliessend 30 Minuten mit strömendem Dampf sterilisiert oder bei 120 °C autoklaviert.

**Patentansprüche für die Vertragsstaaten BE CH DE FR GB IT LI LU NL SE**

1. Phenyl-chinolizidine der allgemeinen Formel

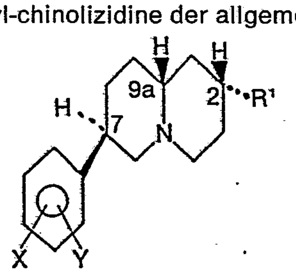

I

worin X, Y und $R^1$ folgendes bedeuten:

X: Wasserstoff, Fluor, Chlor, nieder-Alkoxy, nieder-Alkyl oder Trifluormethyl,

Y: Wasserstoff, Fluor, Chlor, nieder-Alkoxy oder nieder-Alkyl,

$R^1$: Acyl, Acylamino, das durch nieder-Alkyl oder gegebenenfalls durch Halogen, nieder-Alkyl oder nieder-Alkoxy substituiertes Phenyl am Stickstoffatom weiter substituiert sein kann, oder eine Gruppe A mit der Bedeutung

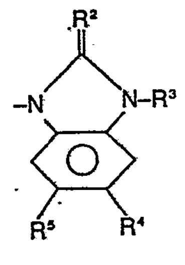

oder

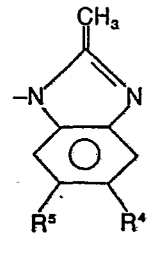

A

mit folgender Bedeutung für $R^2$–$R^5$:

$R^2$: Sauerstoff oder Schwefel,

$R^3$: Wasserstoff oder nieder-Alkyl,

$R^4$ und $R^5$: einer ist Wasserstoff, der andere ist Brom, Jod, Cyan, nieder-Alkoxycarbonyl oder Sulfamoyl,

in Form des Racemats oder der Enantiomeren, sowie Säureadditionssalze solcher Verbindungen, wobei die als nieder bezeichneten Gruppen 1–6 Kohlenstoffatome aufweisen und die als Acyl bezeichneten Reste nieder-Alkanoyl, $C_4$–$C_7$-Cycloalkylcarbonyl, Benzoyl, welches gegebenenfalls durch Halogen, Amino, nieder-Alkoxy, Sulfamoyl und/oder nieder-Alkylsulfonyl substituiert ist, 2-Thiophencarbonyl, 2-Furancarbonyl oder 2-Oxo-1-pyrrolidincarbonyl und die als Acylamino bezeichneten Reste nieder-Alkanoylamino, $C_4$–$C_7$-Cycloalkylcarbonylamino, 2-Thiophencarbonylamino, 2-Furancarbonylamino oder 2-Oxo-1-pyrrolidincarbonylamino bedeuten.

2. Verbindungen gemäss Anspruch 1 der allgemeinen Formel

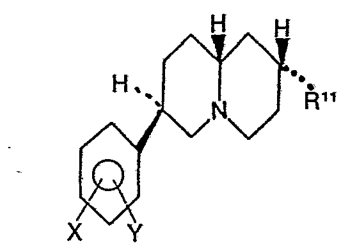

II

worin X und Y dasselbe wie in Anspruch 1 bedeuten und $R^{11}$ Acylamino, das durch nieder-Alkyl oder gegebenenfalls durch Halogen, nieder-Alkyl oder nieder-Alkoxy substituiertes Phenyl am Stickstoffatom weiter substituiert sein kann, darstellt,

in Form des Racemats oder der Enantiomeren, sowie Säureadditionssalze solcher Verbindungen.

3. Verbindungen gemäss Anspruch 2 der allgemeinen Formel

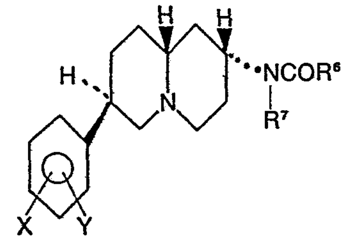

II-I

worin X und Y dasselbe wie in Anspruch 1 bedeuten, $R^6$ nieder-Alkyl und $R^7$ nieder-Alkyl oder Phenyl, das durch Halogen, nieder-Alkyl oder nieder-Alkoxy substituiert sein kann, darstellen,

in Form des Racemats oder der Enantiomeren, sowie Säureadditionssalze solcher Verbindungen.

4. Verbindungen gemäss Anspruch 2, worin Y o-Fluor oder o-Chlor, X Wasserstoff und $R^{11}$ Propionanilido, Cyclopropancarboxamido, 2-Thiophencarboxamido oder 2-Oxo-1-pyrrolidincarboxamido darstellt.

5. Verbindungen gemäss Anspruch 2, worin Y o-Fluor oder o-Chlor, X Wasserstoff und $R^{11}$ 3′-Chlorpropionanilido, 4′-Chlor-propionanilido oder 4′-Fluor-propionanilido darstellt.

6. Verbindungen gemäss Anspruch 2, worin X und Y Wasserstoff und $R^{11}$ Propionanilido oder 4′-Fluor-propionanilido darstellt.

7. rac-N-[(9aβH)-7β-(o-Chlorphenyl)-octahydro-2H-chinolizin-2α-yl]cyclopropancarboxamid

in Form des Racemats oder der Enantiomeren, sowie Säureadditionssalze solcher Verbindungen.

8. rac-N-[(9aβH)-7β-(o-Chlorphenyl)-octahydro-2H-chinolizin-2α-yl]-2-thiophencarboxamid

in Form des Racemats oder der Enantiomeren, sowie Säureadditionssalze solcher Verbindungen.

9. rac-N-[(9aβH)-7β-(o-Chlorphenyl)-octahydro-2H-chinolizin-2α-yl]-2-oxo-1-pyrrolidincarboxamid

in Form des Racemats oder der Enantiomeren, sowie Säureadditionssalze solcher Verbindungen.

10. rac-N-[(9aβH)-7β-(o-chlorphenyl)-octahydro-2H-chinolizin-2α-yl]propionanilid

in Form des Racemats oder der Enantiomeren, sowie Säureadditionssalze solcher Verbindungen.

11. rac-4′-Chlor-N-[(9aβH)-7β-(o-chlorphenyl)-octahydro-2H-chinolizin-2α-yl]propionanilid

in Form des Racemats oder der Enantiomeren, sowie Säureadditionssalze solcher Verbindungen.

12. rac-3′-Chlor-N-[(9aβH)-7β-(o-chlorphenyl)-octahydro-2H-chinolizin-2α-yl]-propionanilid

in Form des Racemats oder der Enantiomeren, sowie Säureadditionssalze solcher Verbindungen.

13. rac-N-(9aβH)-7β-(o-Fluorphenyl)-octahydro-2H-chinolizin-2α-yl)-propionanilid

in Form des Racemats oder der Enantiomeren, sowie Säureadditionssalze solcher Verbindungen.

14. rac-4′-Fluor-N-[(9aβH)-7β-(o-fluorphenyl)-octahydro-2H-chinolizin-2α-yl]-propionanilid
in Form des Racemats oder der Enantiomeren, sowie Säureadditionssalze solcher Verbindungen.

15. rac-4′-Fluor-N-[(9aβH)-7β-phenyloctahydro-2H-chinolizin-2α-yl]-propionanilid
in Form des Racemats oder der Enantiomeren, sowie Säureadditionssalze solcher Verbindungen.

16. rac-N-[(9aβH)-7β-(o-Fluorphenyl)-octahydro-2H-chinolizin-2α-yl]-acetanilid
in Form des Racemats oder der Enantiomeren, sowie Säureadditionssalze solcher Verbindungen.

17. rac-N-[(9aβH)-7β-(o-Chlorphenyl)-octahydro-2H-chinolizin-2α-yl]-N-butylpropionamid
in Form des Racemats oder der Enantiomeren, sowie Säureadditionssalze solcher Verbindungen.

18. rac-N-[(9aβH)-7β-Phenyl-octahydro-2H-chinolizin-2α-yl]-propionanilid
in Form des Racemats oder der Enantiomeren, sowie Säureadditionssalze solcher Verbindungen.

19. Verbindungen gemäss Anspruch 1 der allgemeinen Formel

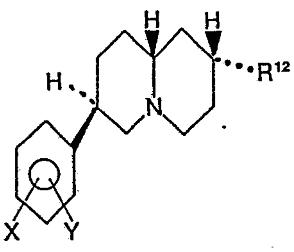

III

worin X und Y dasselbe wie in Anspruch 1 bedeuten und $R^{12}$ Acyl darstellt,
in Form des Racemats oder der Enantiomeren, sowie Säureadditionssalze solcher Verbindungen.

20. Verbindungen gemäss Anspruch 19, worin Y o-Fluor oder o-Chlor, X Wasserstoff und $R^{12}$ p-Fluorbenzoyl darstellen.

21. rac-(9aβH)-7β-(o-Chlorphenyl)octahydro-2H-chinolizin-2α-yl)p-fluorphenylketon
in Form des Racemats oder der Enantiomeren, sowie Säureadditionssalze solcher Verbindungen.

22. Verbindungen gemäss Anspruch 1 der allgemeinen Formel

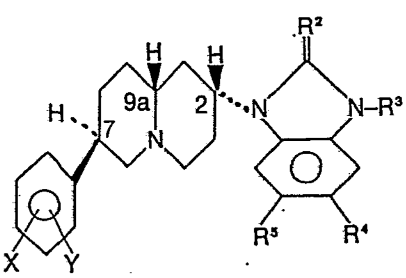

IV–1

worin X, Y sowie $R^2$–$R^5$ dasselbe wie in Anspruch 1 bedeuten,
in Form des Racemats oder der Enantiomeren, sowie Säureadditionssalze solcher Verbindungen.

23. rac-1-[(9aβH)-7β-(o-Fluorphenyl)-octahydro-2H-chinolizin-2α-yl]-5-brom-2-benzimidazolinon
in Form des Racemats oder der Enantiomeren, sowie Säureadditionssalze solcher Verbindungen.

24. rac-1-[(9aβH)-7β-(o-Fluorphenyl)-octahydro-2H-chinolizin-2α-yl]-5-jod-2-benzimidazolinon
in Form des Racemats oder der Enantiomeren, sowie Säureadditionssalze solcher Verbindungen.

25. rac-1-[(9aβH)-7β-(o-Fluorphenyl)-octahydro-2H-chinolizin-2α-yl]-5-cyan-2-benzimidazolinon
in Form des Racemats oder der Enantiomeren, sowie Säureadditionssalze solcher Verbindungen.

26. Verbindungen gemäss Anspruch 1 der allgemeinen Formel

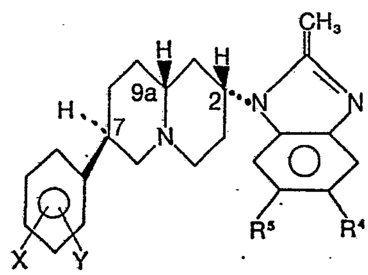

IV–2

worin X, Y, $R^4$ und $R^5$ dasselbe wie in Anspruch 1 bedeuten,
in Form des Racemats oder der Enantiomeren, sowie Säureadditionssalze solcher Verbindungen.

27. Ungesättigte Phenyl-chinolizidine der allgemeinen Formel

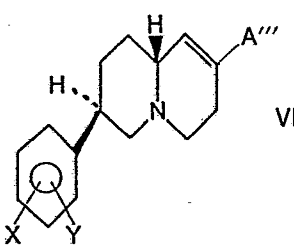

VII+XIII

worin X und Y dasselbe wie in Anspruch 1 bedeuten und A‴ wie A in Anspruch 1 ist, wobei, falls $R^3$ nieder-Alkyl bedeutet, $R^2$ Sauerstoff darstellt, in Form des Racemats und der Enantiomeren.

28. Phenylendiamine der allgemeinen Formel

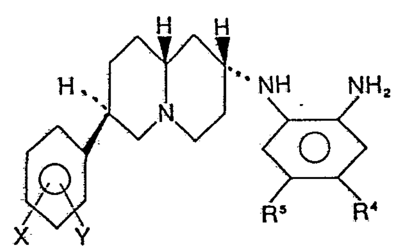

VIII

worin X, Y, $R^4$ und $R^5$ dasselbe wie in Anspruch 1 bedeuten,
in Form des Racemats und der Enantiomeren.

29. Primäre oder sekundäre Amine der allgemeinen Formel

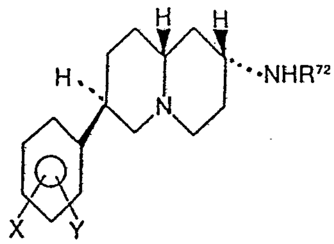

V-1

worin X und Y dasselbe wie in Anspruch 1 bedeuten und $R^{72}$ nieder-Alkyl oder Phenyl, das durch Halogen, nieder-Alkyl oder nieder-Alkoxy substituiert sein kann, darstellt,
in Form des Racemats und der Enantiomeren.

30. Ylidenverbindungen der allgemeinen Formel

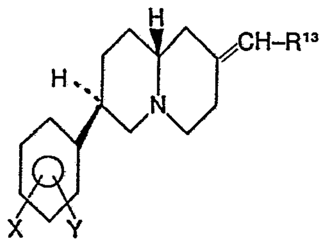

VI

in der X und Y dasselbe wie in Anspruch 1 bedeuten und die Ylidengruppe $=CH-R^{13}$ dem Acylrest $R^{12}$ in Anspruch 19, worin die Oxofunktion durch Wasserstoff ausgetauscht wurde, entspricht,
in Form des Racemats und der Enantiomeren.

31. Phenyl-chinolizidine gemäss einem der Ansprüche 1–17 zur Anwendung in einem therapeutischen Verfahren als pharmakologisch aktive Wirkstoffe, insbesondere als antipsychotische und/oder analgetische Wirkstoffe.

32. Verfahren zur Herstellung von Phenyl-chinolizidinen der allgemeinen Formel I in Anspruch 1 in Form des Racemats oder der Enantiomeren, sowie von Säureadditionssalzen solcher Verbindungen, dadurch gekennzeichnet, dass man

a) zur Herstellung von Carboxamiden der allgemeinen Formel

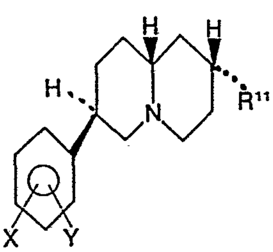

II

worin X und Y dasselbe wie in Anspruch 1 bedeuten und $R^{11}$ Acylamino, das durch nieder-Alkyl oder gegebenenfalls durch Halogen, nieder-Alkyl oder nieder-Alkoxy substituiertes Phenyl am Stickstoffatom weiter substituiert sein kann, darstellt,
ein primäres oder sekundäres Amin der allgemeinen Formel

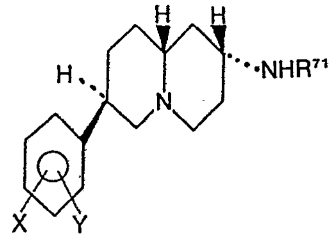

V

worin X und Y dasselbe wie in Anspruch 1 bedeuten und $R^{71}$ Wasserstoff, nieder-Alkyl oder Phenyl, das durch Halogen, nieder-Alkyl oder nieder-Alkoxy substituiert sein kann, darstellt.
acyliert, oder dass man

b) zur Herstellung von Ketonen der allgemeinen Formel

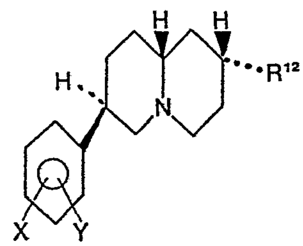

III

in der X und Y dasselbe wie in Anspruch 1 bedeuten und $R^{12}$ Acyl darstellt,
eine Verbindung der allgemeinen Formel

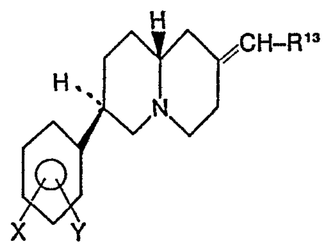

VI

in der X und Y dasselbe wie in Anspruch 1 bedeuten und die Ylidengruppe $=CH-R^{13}$ dem Acylrest $R^{12}$, worin die Oxofunktion durch Wasserstoff ausgetauscht wurde entspricht,
mit Diboran und Wasserstoffperoxid behandelt und das erhaltene Carbinol einer Oxidation unterwirft; oder dass man

c) zur Herstellung einer Verbindung der allgemeinen Formel

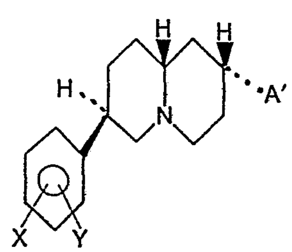

IV-3

worin X und Y dasselbe wie in Anspruch 1 bedeuten und A′ wie A ist, wobei jedoch $R^2$ Sauerstoff darstellt und $R^4$ und $R^5$ von Brom und Jod verschieden sind,
eine Verbindung der allgemeinen Formel

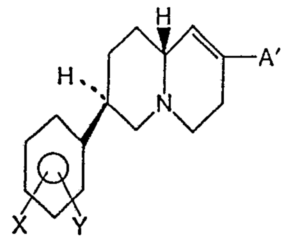

VII

worin X, Y und A′ dasselbe wie in Anspruch 1 bedeuten,
katalytisch hydriert, oder dass man

d) zur Herstellung einer Verbindung der allgemeinen Formel

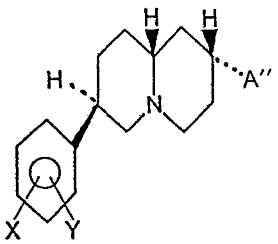

IV–4

worin X und Y dasselbe wie in Anspruch 1 bedeuten und A″ wie A ist, wobei jedoch $R^2$ Sauerstoff und $R^3$ Wasserstoff darstellt,
ein Phenylen-diamin der allgemeinen Formel

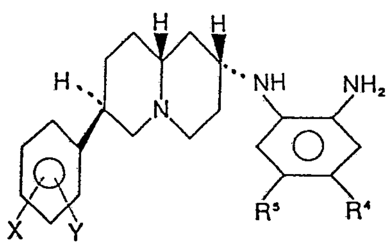

VIII

worin X, Y, $R^4$ und $R^5$ dasselbe wie in Anspruch 1 bedeuten,
mit einem Cyclisierungsmittel der allgemeinen Formel

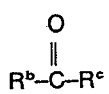

IX

worin $R^b$ und $R^c$ Halogen, Amino oder 1-Imidazolyl bedeuten,
oder mit Essigsäure oder einem Orthoessigsäureester kondensiert, oder dass man

e) zur Herstellung von Verbindungen der allgemeinen Formel

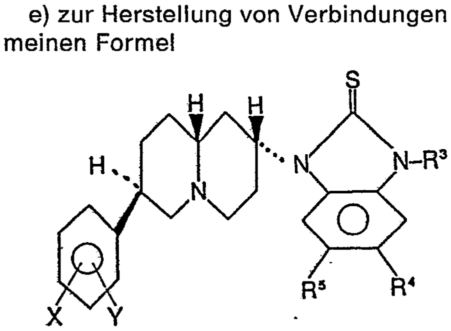

IV–5

worin X, Y sowie $R^3$–$R^5$ dasselbe wie in Anspruch 1 bedeuten,
entweder eine entsprechende Oxo-Verbindung der allgemeinen Formel

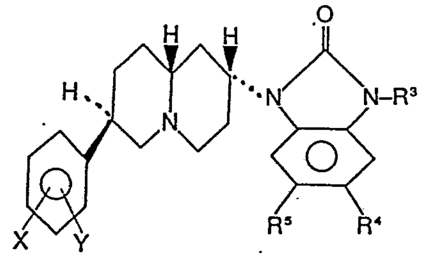

IV–6

worin X, Y sowie $R^3$–$R^5$ dasselbe wie in Anspruch 1 bedeuten,
mit $P_2S_5$ umsetzt, oder ein Phenylen-diamin der obigen allgemeinen Formel VIII mit Thiophosgen, Thiocarbonylidiimidazol oder $CS_2$ umsetzt und anschliessend gegebenenfalls N-alkyliert, oder dass man

f) zur Herstellung von Verbindungen der allgemeinen Formel

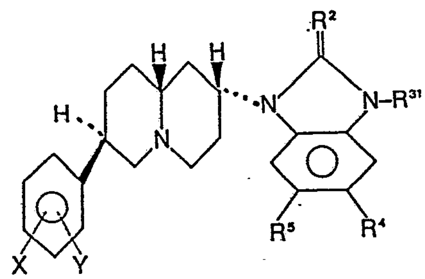

IV–7

worin X, Y, $R^2$, $R^4$ und $R^5$ dasselbe wie in Anspruch 1 bedeuten und $R^{31}$ nieder-Alkyl darstellt,
eine Verbindung der allgemeinen Formel

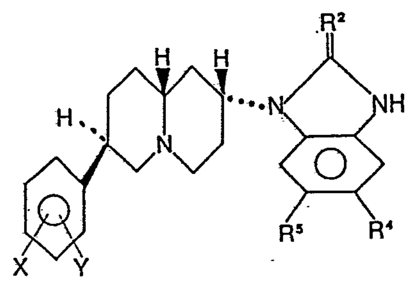

IV–8

worin X, Y, $R^2$, $R^4$ und $R^5$ dasselbe wie in Anspruch 1 bedeuten,
N-alkyliert, wonach man erhaltene Racemate gegebenenfalls spaltet und erhaltene Basen gegebenenfalls in Säureadditionssalze überführt.

33. Arzneimittel, insbesondere zur Verwendung als Antipsychotica und/oder Analgetica, enthaltend ein Phenylchinolizidin der vorstehend angegebenen Formel I in Form des Racemats oder eines Enantiomeren oder eines Säureadditionssalzes einer solchen Verbindung.

**Patentansprüche für den Vertragsstaat AT**

1. Verfahren zur Herstellung von Phenyl-chinolizidinen der allgemeinen Formel

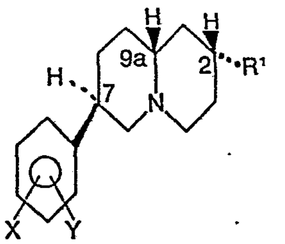

I

worin X, Y und $R^1$ folgendes bedeuten:

X: Wasserstoff, Fluor, Chlor, nieder-Alkoxy, nieder-Alkyl oder Trifluormethyl,

Y: Wasserstoff, Fluor, Chlor, nieder-Alkoxy oder nieder-Alkyl,

$R^1$: Acyl, Acylamino, das durch nieder-Alkyl oder gegebenenfalls durch Halogen, nieder-Alkyl oder nieder-Alkoxy substituiertes Phenyl am Stickstoffatom weiter substituiert sein kann, oder eine Gruppe A mit der Bedeutung

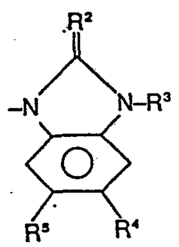

oder

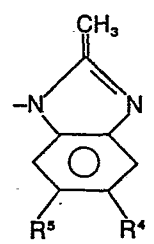

A

mit folgender Bedeutung für $R^2$–$R^5$:

$R^2$: Sauerstoff oder Schwefel,

$R^3$: Wasserstoff oder nieder-Alkyl,

$R^4$ und $R^5$: einer ist Wasserstoff, der andere ist Brom, Jod, Cyan, nieder-Alkoxycarbonyl oder Sulfamoyl, wobei die als nieder bezeichneten Gruppen 1–6 Kohlenstoffatome aufweisen und die als Acyl bezeichneten Reste nieder-Alkanoyl, $C_4$–$C_7$-Cycloalkylcarbonyl, Benzoyl, welches gegebenenfalls durch Halogen, Amino, nieder-Alkoxy, Sulfamoyl und/oder nieder-Alkylsulfonyl substituiert ist, 2-Thiophencarbonyl, 2-Furancarbonyl oder 2-Oxo-1-pyrrolidincarbonyl und die als Acylamino bezeichneten Reste nieder-Alkanoylamino, $C_4$–$C_7$-Cycloalkylcarbonylamino, 2-Thiophencarbonylamino, 2-Furancarbonylamino oder 2-Oxo-1-pyrrolidincarbonylamino bedeuten,
in Form des Racemats oder der Enantiomeren, sowie von Säureadditionssalzen solcher Verbindungen, dadurch gekennzeichnet, dass man

a) zur Herstellung von Carboxamiden der allgemeinen Formel

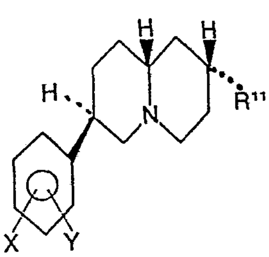

II

worin X und Y dasselbe wie oben bedeuten und $R^{11}$ Acylamino, das durch nieder-Alkyl oder gegebenenfalls durch Halogen, nieder-Alkyl oder nieder-Alkoxy substituiertes Phenyl am Stickstoffatom weiter substituiert sein kann, darstellt,
ein primäres oder sekundäres Amin der allgemeinen Formel

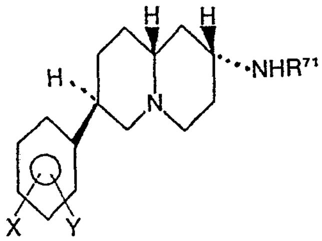

V

worin X und Y dasselbe wie oben bedeuten und $R^{71}$ Wasserstoff, nieder-Alkyl oder Phenyl, das durch Halogen, nieder-Alkyl oder nieder-Alkoxy substituiert sein kann, darstellt,
acyliert, oder dass man

b) zur Herstellung von Ketonen der allgemeinen Formel

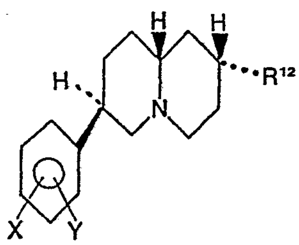

III

in der X und Y dasselbe wie oben bedeuten und $R^{12}$ Acyl darstellt,
eine Verbindung der allgemeinen Formel

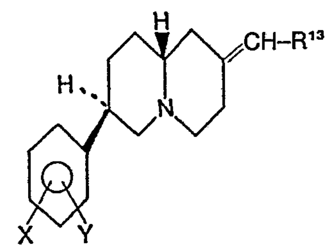

VI

in der X und Y dasselbe wie oben bedeuten und die Ylidengruppe $=CH-R^{13}$ dem Acylrest $R^{12}$, worin die Oxofunktion durch Wasserstoff ausgetauscht wurde, entspricht,
mit Diboran und Wasserstoffperoxid behandelt und das erhaltene Carbinol einer Oxidation unterwirft; oder dass man

c) zur Herstellung einer Verbindung der allgemeinen Formel

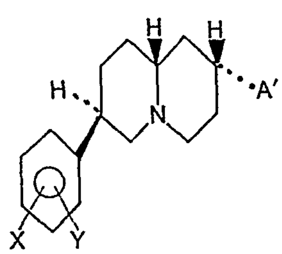

IV–3

worin X und Y dasselbe wie oben bedeuten und A′ wie A ist, wobei jedoch $R^2$ Sauerstoff darstellt und

$R^4$ und $R^5$ von Brom und Jod verschieden sind, eine Verbindung der allgemeinen Formel

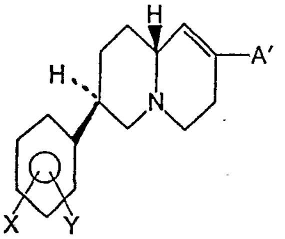

VII

worin X, Y und A′ dasselbe wie oben bedeuten, katalytisch hydriert, oder dass man

d) zur Herstellung einer Verbindung der allgemeinen Formel

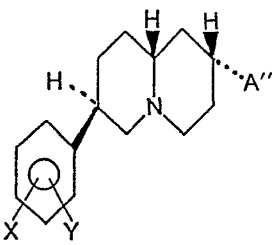

IV–4

worin X und Y dasselbe wie oben bedeuten und A″ wie A ist, wobei jedoch $R^2$ Sauerstoff und $R^3$ Wasserstoff darstellt,
ein Phenylen-diamin der allgemeinen Formel

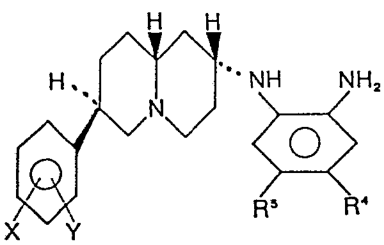

VIII

worin X, Y, $R^4$ und $R^5$ dasselbe wie oben bedeuten, mit einem Cyclisierungsmittel der allgemeinen Formel

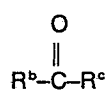

IX

worin $R^b$ und $R^c$ Halogen, Amino oder 1-Imidazolyl bedeuten,
oder mit Essigsäure oder einem Orthoessigsäureester kondensiert, oder dass man

e) zur Herstellung von Verbindungen der allgemeinen Formel

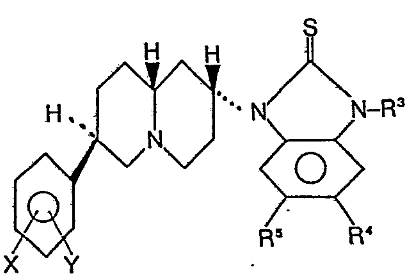

IV–5

worin X, Y sowie $R^3$–$R^5$ dasselbe wie oben bedeuten,
entweder eine entsprechende Oxo-Verbindung der allgemeinen Formel

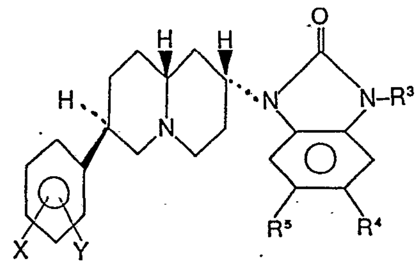

IV–6

worin X, Y sowie $R^3$–$R^5$ dasselbe wie oben bedeuten,
mit $P_2S_5$ umsetzt, oder ein Phenylen-diamin der obigen allgemeinen Formel VIII mit Thiophosgen, Thiocarbonyldiimidazol oder $CS_2$ umsetzt und anschliessend gegebenenfalls N-alkyliert, oder dass man

f) zur Herstellung von Verbindungen der allgemeinen Formel

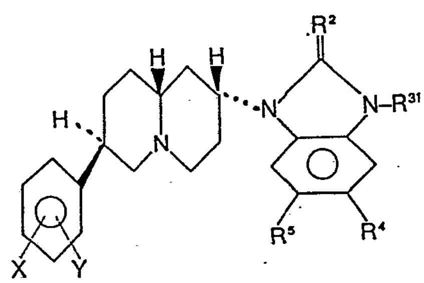

IV–7

worin X, Y, $R^2$, $R^4$ und $R^5$ dasselbe wie oben bedeuten und $R^{31}$ nieder-Alkyl darstellt,
eine Verbindung der allgemeinen Formel

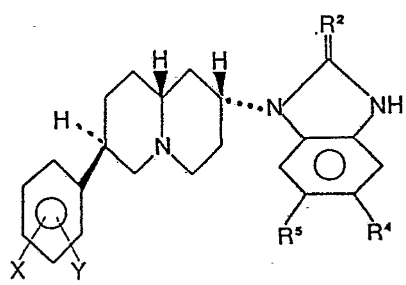

IV–8

worin X, Y, $R^2$, $R^4$ und $R^5$ dasselbe wie oben bedeuten,
N-alkyliert, wonach man erhaltene Racemate gegebenenfalls spaltet und erhaltene Basen gegebenenfalls in Säureadditionssalze überführt.

2. Verfahren nach Anspruch 1 zur Herstellung von Verbindungen der allgemeinen Formel

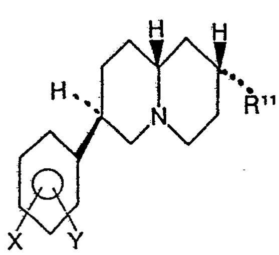

II

worin X und Y dasselbe wie in Anspruch 1 bedeuten und $R^{11}$ Acylamino, das durch nieder-Alkyl

oder gegebenenfalls durch Halogen, nieder-Alkyl oder nieder-Alkoxy substituiertes Phenyl am Stickstoffatom weiter substituiert sein kann, darstellt,
in Form des Racemats oder der Enantiomeren sowie von Säureadditionssalzen solcher Verbindungen, dadurch gekennzeichnet, dass man entsprechend substituierte Ausgangsverbindungen einsetzt.

3. Verfahren nach Anspruch 2 zur Herstellung von Verbindungen der allgemeinen Formel

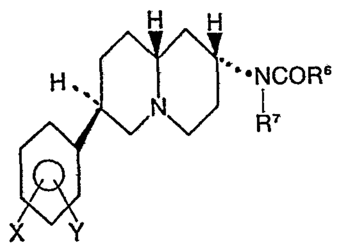

II–I

worin X und Y dasselbe wie in Anspruch 1 bedeuten, $R^6$ nieder-Alkyl und $R^7$ nieder-Alkyl oder Phenyl, das durch Halogen, nieder-Alkyl oder nieder-Alkoxy substituiert sein kann, darstellen,
in Form des Racemats oder der Enantiomeren sowie von Säureadditionssalzen solcher Verbindungen, dadurch gekennzeichnet, dass man entsprechend substituierte Ausgangsverbindungen einsetzt.

4. Verfahren nach Anspruch 2 zur Herstellung von Verbindungen, worin Y o-Fluor oder o-Chlor, X Wasserstoff und $R^{11}$ Propionanilido, Cyclopropancarboxamido, 2-Thiophencarboxamido oder 2-Oxo-1-pyrrolidincarboxamido darstellt, dadurch gekennzeichnet, dass man entsprechend substituierte Ausgangsverbindungen einsetzt.

5. Verfahren nach Anspruch 2 zur Herstellung von Verbindungen, worin Y o-Fluor oder o-Chlor, X Wasserstoff und $R^{11}$ 3′-Chlor-propionanilido, 4′-Chlor-propionanilido oder 4′-Fluor-propionanilido darstellt, dadurch gekennzeichnet, dass man entsprechend substituierte Ausgangsverbindungen einsetzt.

6. Verfahren nach Anspruch 2 zur Herstellung von Verbindungen, worin X und Y Wasserstoff und $R^{11}$ Propionanilido oder 4′-Fluorpropionanilido darstellt.

7. Verfahren nach Anspruch 2 zur Herstellung von
rac-N-[(9aβH)-7β-(o-Chlorphenyl)octahydro-2H-chinolizin-2α-yl]cyclopropancarboxamid
in Form des Racemats oder der Enantiomeren, sowie von Säureadditionssalzen solcher Verbindungen, dadurch gekennzeichnet, dass man entsprechend substituierte Ausgangsverbindungen einsetzt.

8. Verfahren nach Anspruch 2 zur Herstellung von
rac-N-[(9aβH)-7β-(o-Chlorphenyl)octahydro-2H-chinolizin-2α-yl]-2-thiophencarboxamid
in Form des Racemats oder der Enantiomeren, sowie von Säureadditionssalzen solcher Verbindungen, dadurch gekennzeichnet, dass man entsprechend substituierte Ausgangsverbindungen einsetzt.

9. Verfahren nach Anspruch 2 zur Herstellung von
rac-N-[(9aβH)-7β-(o-Chlorphenyl)octahydro-2H-chinolizin-2α-yl]-2-oxo-1-pyrrolidincarboxamid
in Form des Racemats oder der Enantiomeren, sowie von Säureadditionssalzen solcher Verbindungen, dadurch gekennzeichnet, dass man entsprechend substituierte Ausgangsverbindungen einsetzt.

10. Verfahren nach Anspruch 2 zur Herstellung von
rac-N-[(9aβH)-7β-(o-Chlorphenyl)octahydro-2H-chinolizin-2α-yl]propionanilid
in Form des Racemats oder der Enantiomeren, sowie Säureadditionssalze solcher Verbindungen, dadurch gekennzeichnet, dass man entsprechend substituierte Ausgangsverbindungen einsetzt.

11. Verfahren nach Anspruch 2 zur Herstellung von
rac-4′-Chlor-N-[(9aβH)-7β-(o-chlorphenyl)-octahydro-2H-chinolizin-2α-yl]propionanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

12. Verfahren nach Anspruch 2 zur Herstellung von
rac-3′-Chlor-N-[(9aβH)-7β-(o-chlorphenyl)-octahydro-2H-chinolizin-2α-yl]-propionanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

13. Verfahren nach Anspruch 2 zur Herstellung von
rac-N-[(9aβH)-7β-(o-Fluorphenyl)-octahydro-2H-chinolizin-2α-yl]-propionanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

14. Verfahren nach Anspruch 2 zur Herstellung von
rac-4′-Fluor-N-[(9aβH)-7β-(o-fluorphenyl)-octahydro-2H-chinolizin-2α-yl]-propionanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

15. Verfahren nach Anspruch 2 zur Herstellung von
rac-4′-Fluor-N-[(9aβH)-7β-phenyl-octahydro-2H-chinolizin-2α-yl]-propionanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

16. Verfahren nach Anspruch 2 zur Herstellung von
rac-N-[(9aβH)-7β-(o-Fluorphenyl)-octahydro-2H-chinolizin-2α-yl]-acetanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

17. Verfahren nach Anspruch 2 zur Herstellung von
rac-N-[(9aβH)-7β-(o-Chlorphenyl)-octahydro-2H-chinolizin-2α-yl]-N-butylpropionamid
in Form des Racemats oder der Enantiomeren, sowie von Säureadditionssalzen solcher Verbindungen, dadurch gekennzeichnet, dass man entsprechend substituierte Ausgangsverbindungen einsetzt.

18. Verfahren nach Anspruch 2 zur Herstellung von
rac-N-[(9aβH)-7β-Phenyl-octahydro-2H-chinolizin-2α-yl]-propionanilid
in Form des Racemats oder der Enantiomeren, sowie von Säureadditionssalzen solcher Verbindungen, dadurch gekennzeichnet, dass man entsprechend substituierte Ausgangsverbindungen einsetzt.

19. Verfahren nach Anspruch 1 zur Herstellung von Verbindungen der allgemeinen Formel

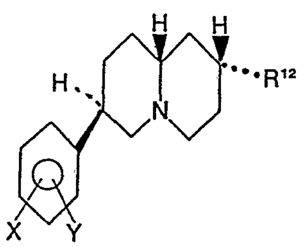

III

worin X und Y dasselbe wie in Anspruch 1 bedeuten und $R^{12}$ Acyl darstellt,
in Form des Racemats oder der Enantiomeren sowie von Säureadditionssalzen solcher Verbindungen, dadurch gekennzeichnet, dass man entsprechend substituierte Ausgangsverbindungen einsetzt.

20. Verfahren nach Anspruch 19 zur Herstellung von Verbindungen, worin Y o-Fluor oder o-Chlor, X Wasserstoff und $R^{12}$ p-Fluorbenzoyl darstellen, dadurch gekennzeichnet, dass man entsprechend substituierte Ausgangsverbindungen einsetzt.

21. Verfahren nach Anspruch 20 zur Herstellung von
rac-(9aβH)-7β-(o-Chlorphenyl)octahydro-2H-chinolizin-2α-yl)-p-fluorphenylketon
in Form des Racemats oder der Enantiomeren, sowie von Säureadditionssalzen solcher Verbindungen, dadurch gekennzeichnet, dass man entsprechend substituierte Ausgangsverbindungen einsetzt.

22. Verfahren nach Anspruch 1 zur Herstellung von Verbindungen der allgemeinen Formel

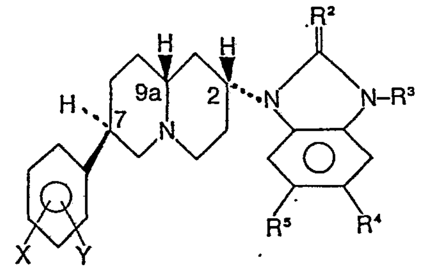

IV–1

worin X, Y sowie $R^2$–$R^5$ dasselbe wie in Anspruch 1 bedeuten,
in Form des Racemats oder der Enantiomeren, sowie von Säureadditionssalzen solcher Verbindungen, dadurch gekennzeichnet, dass man entsprechend substituierte Ausgangsverbindungen einsetzt.

23. Verfahren nach Anspruch 22 zur Herstellung von
rac-1-[(9aβH)-7β-(o-Fluorphenyl)-octahydro-2H-chinolizin-2α-yl]-5-brom-2-benzimidazolinon
in Form des Racemats oder der Enantiomeren, sowie von Säureadditionssalzen solcher Verbindungen, dadurch gekennzeichnet, dass man entsprechend substituierte Ausgangsverbindungen einsetzt.

24. Verfahren nach Anspruch 22 zur Herstellung von
rac-1-[(9aβH)-7β-(o-Fluorphenyl)-octahydro-2H-chinolizin-2α-yl]-5-jod-2-benzimidazolinon
in Form des Racemats oder der Enantiomeren, sowie von Säureadditionssalzen solcher Verbindungen, dadurch gekennzeichnet, dass man entsprechend substituierte Ausgangsverbindungen einsetzt.

25. Verfahren nach Anspruch 22 zur Herstellung von
rac-1-[(9aβH)-7β-(o-Fluorphenyl)-octahydro-2H-chinolizin-2α-yl]-5-cyan-2-benzimidazolinon
in Form des Racemats oder der Enantiomeren, sowie von Säureadditionssalzen solcher Verbindungen, dadurch gekennzeichnet, dass man entsprechend substituierte Ausgangsverbindungen einsetzt.

26. Verfahren nach Anspruch 1 zur Herstellung von Verbindungen der allgemeinen Formel

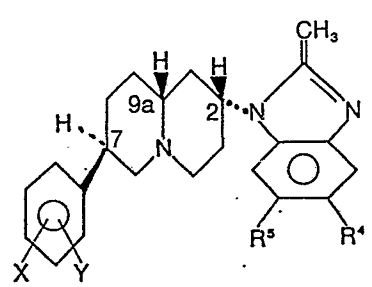

IV–2

worin X, Y, $R^4$ und $R^5$ dasselbe wie in Anspruch 1 bedeuten,
in Form des Racemats oder der Enantiomeren, sowie von Säureadditionssalzen solcher Verbindungen, dadurch gekennzeichnet, dass man entsprechend substituierte Ausgangsverbindungen einsetzt.

**Claims for the Contracting states: BE, CH, DE, FR, GB, IT, LI, LU, NL, SE**

1. Phenyl-quinolizidines of the general formula

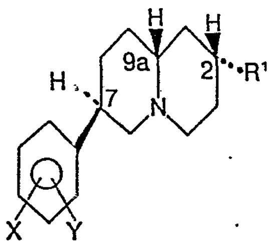

I

wherein X, Y and $R^1$ signify the following:

X: hydrogen, fluorine, chlorine, lower-alkoxy, lower-alkyl or trifluormethyl,

Y: hydrogen, fluorine, chlorine, lower-alkoxy or lower-alkyl,

$R^1$: alcyl, acylamino, which can be further substituted on the nitrogen atom by lower-alkyl or phenyl optionally substituted by halogen, lower-alkyl or lower-alkoxy, or a group A with the significance

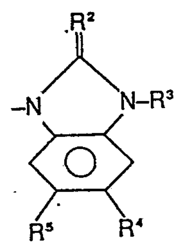

or

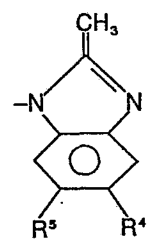

A

with the following significance for $R^2$–$R^5$:

$R^2$: oxygen or sulphur,

$R^3$: hydrogen or lower-alkyl,

$R^4$ and $R^5$: one is hydrogen, the other is bromine, iodine, cyano, lower-alkoxycarbonyl or sulphamoyl,

in the form of the racemate or of the enantiomers, as well as acid addition salts of such compounds, whereby the groups denoted as lower have 1–6 carbon atoms and the residues denoted as acyl signify lower-alkanoyl, $C_4$–$C_7$-cycloalkyl-carbonyl, benzoyl, which is optionally substituted by halogen, amino, lower-alkoxy, sulphamoyl and/or lower-alkyl-sulphonyl, 2-thiophenecarbonyl, 2-furancarbonyl or 2-oxo-1-pyrrolidinecarbonyl and the residues denoted as acylamino signify lower-alkanoylamino, $C_4$–$C_7$-cycloalkylcarbonylamino, 2-thiophenecarbonylamino, 2-furancarbonylamino or 2-oxo-1-pyrrolidinecarbonylamino.

2. Compounds in accordance with claim 1 of the general formula

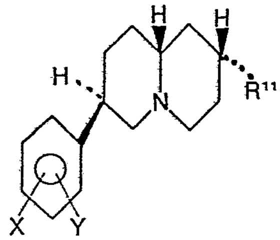

II

wherein X and Y signify the same as in claim 1 and $R^{11}$ represents acylamino, which can be further substituted on the nitrogen atom by lower-alkyl or phenyl optionally substituted by halogen, lower-alkyl or lower-alkoxy,

in the form of the racemate or of the enantiomers, as well as acid addition salts of such compounds.

3. Compounds in accordance with claim 2 of the general formula

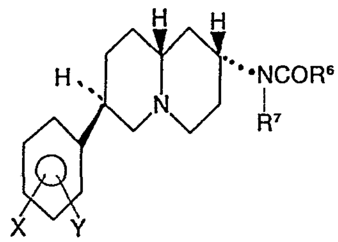

II–I

wherein X and Y signify the same as in claim 1, $R^6$ represents lower-alkyl and $R^7$ represents lower-alkyl or phenyl, which can be substituted by halogen, lower-alkyl or lower-alkoxy,

in the form of the racemate or of the enantiomers, as well as acid addition salts of such compounds.

4. Compounds in accordance with claim 2, wherein Y represents o-fluoro or o-chloro, X represents hydrogen and $R^{11}$ represents propionanilido, cyclopropanecarboxamido, 2-thiophenecarboxamido or 2-oxo-1-pyrrolidinecarboxamido.

5. Compounds in accordance with claim 2, wherein Y represents o-fluoro or o-chloro, X represents hydrogen and $R^{11}$ represents 3′-chloro-propionanilido, 4′-chloro-propionanilido or 4′-fluoro-propionanilido.

6. Compounds in accordance with claim 2, wherein X and Y represent hydrogen and $R^{11}$ represents propionanilido or 4′-fluoro-propionanilido.

7. rac-N-[(9aβH)-7β-(o-Chlorophenyl)octahydro-2H-quinolizin-2α-yl]cyclopropanecarboxamide

in the form of the racemate or of the enantiomers, as well as acid addition salts of such compounds.

8. rac-N-[(9aβH)-7β-(o-Chlorophenyl)octahydro-2H-quinolizin-2α-yl]-2-thiophenecarboxamide

in the form of the racemate or of the enantiomers, as well as acid addition salts of such compounds.

9. rac-N-[(9aβH)-7β-(o-Chlorophenyl)octahydro-2H-quinolizin-2α-yl]-2-oxo-1-pyrrolidinecarboxamide

in the form of the racemate or of the enantiomers, as well as acid addition salts of such compounds.

10. rac-N-[(9aβH)-7β-(o-Chlorophenyl)octahydro-2H-quinolizin-2α-yl]propionanilide

in the form of the racemate or of the enantiomers, as well as acid addition salts of such compounds.

11. rac-4′-Chloro-N-[(9aβH)-7β-(o-chlorophenyl)octahydro-2H-quinolizin-2α-yl]-propionanilide

in the form of the racemate or of the enantiomers, as well as acid addition salts of such compounds.

12. rac-3′-Chloro-N-[(9aβH)-7β-(o-chlorophenyl)-octahydro-2H-quinolizin-2α-yl]-propionanilide

in the form of the racemate or of the enantiomers, as well as acid addition salts of such compounds.

13. rac-N-(9aβH)-7β-(o-Fluorophenyl)octahydro-2H-quinolizin-2α-yl)-propionanilide

in the form of the racemate or of the enantiomers, as well as acid addition salts of such compounds.

14. rac-4′-Fluoro-N-[(9aβH)-7β-(o-fluorophenyl)-octahydro-2H-quinolizin-2α-yl]-propionanilide

in the form of the racemate or of the enantiomers, as well as acid addition salts of such compounds.

15. rac-4′-Fluoro-N-[(9aβH)-7β-phenyloctahydro-2H-quinolizin-2α-yl]-propionanilide

in the form of the racemate or of the enantiomers, as well as acid addition salts of such compounds.

16. rac-N-[(9aβH)-7β-(o-Fluorophenyl)octahydro-2H-quinolizin-2α-yl]-acetanilide

in the form of the racemate or of the enantiomers, as well as acid addition salts of such compounds.

17. rac-N-[(9aβH)-7β-(o-Chlorophenyl)-octahydro-2H-quinolizin-2α-yl]-N-butylpropionamide

in the form of the racemate or of the enantiomers, as well as acid addition salts of such compounds.

18. rac-N-[(9aβH)-7β-Phenyl-octahydro-2H-quinolizin-2α-yl]-propionanilide

in the form of the racemate or of the enantiomers, as well as acid addition salts of such compounds.

19. Compounds in accordance with claim 1 of the general formula

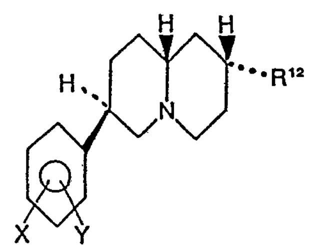

III

wherein X and Y signify the same as in claim 1 and $R^{12}$ represents acyl,

in the form of the racemate or of the enantiomers, as well as acid addition salts of such compounds.

20. Compounds in accordance with claim 19, wherein Y represents o-fluoro or o-chloro, X represents hydrogen and $R^{12}$ represents p-fluorobenzoyl.

21. rac-(9aβH)-7β-(o-Chlorophenyl)octahydro-2H-quinolizin-2α-yl) p-fluorophenyl ketone

in the form of the racemate or of the enantiomers, as well as acid addition salts of such compounds.

22. Compounds in accordance with claim 1 of the general formula

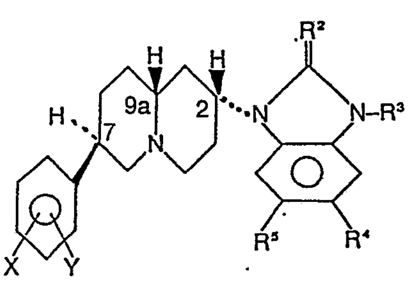

IV–1

wherein X, Y as well as $R^2$–$R^5$ signify the same as in claim 1,

in the form of the racemate or of the enantiomers, as well as acid addition salts of such compounds.

23. rac-1-[(9aβH)-7β-(o-fluorophenyl)-octahydro-2H-quinolizin-2α-yl]-5-bromo-2-benzimidazolinone

in the form of the racemate or of the enantiomers, as well as acid addition salts of such compounds.

24. rac-1-[(9aβH)-7β-(o-Fluorophenyl)-octahydro-2H-quinolizin-2α-yl]-5-iodo-2-benzimidazolinone

in the form of the racemate or of the enantiomers, as well as acid addition salts of such compounds.

25. rac-1-[(9aβH)-7β-(o-Fluorophenyl)-octahydro-2H-quinolizin-2α-yl]-5-cyano-2-benzimidazolinone

in the form of the racemate or of the enantiomers, as well as acid addition salts of such compounds.

26. Compounds in accordance with claim 1 of the general formula

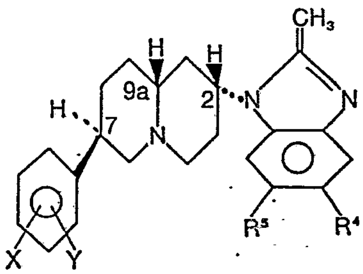

IV–2

wherein X, Y, $R^4$ and $R^5$ signify the same as in claim 1,

in the form of the racemate or of the enantiomers, as well as acid addition salts of such compounds.

27. Unsaturated phenyl-quinolizidines of the general formula

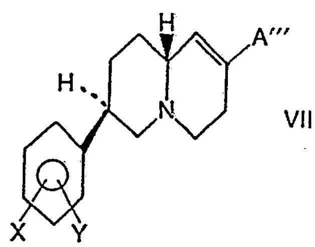

VII+XIII

wherein X and Y signify the same as in claim 1 and A‴ is as A in claim 1, whereby $R^2$ represents oxygen when $R^3$ signifies lower-alkyl,

in the form of the racemate and of the enantiomers.

28. Phenylenediamines of the general formula

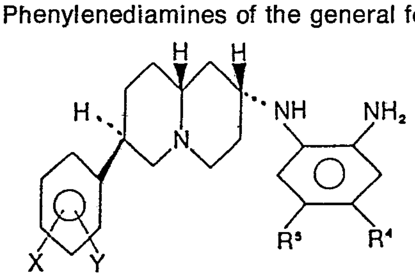

VIII

wherein X, Y, $R^4$ and $R^5$ signify the same as in claim 1,

in the form of the racemate and of the enantiomers.

29. Primary or secondary amines of the general formula

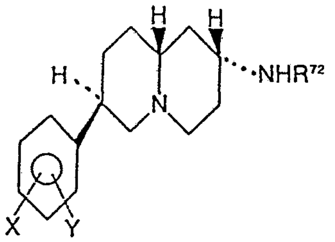

V–1

wherein X and Y signify the same as in claim 1 and $R^{72}$ represents lower-alkyl or phenyl, which can be substituted by halogen, lower-alkyl or lower-alkoxy,
in the form of the racemate and of the enantiomers.

30. Ylidene compounds of the general formula

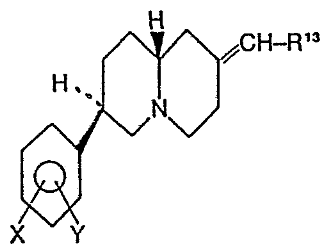

VI

in which X and Y signify the same as in claim 1 and the ylidene group $=CH-R^{13}$ corresponds to the acyl residue $R^{12}$ in claim 19 in which the oxo function has been replaced by hydrogen,
in the form of the racemate and of the enantiomers.

31. Phenyl-quinolizidines in accordance with any one of claims 1–17 for use in a therapeutic procedure as pharmacologically active substances, especially as antipsychotic and/or analgesic active substances.

32. A process for the manufacture of phenyl-quinolizidines of general formula I in claim 1 in the form of the racemate or of the enantiomers, as well as of acid addition salts of such compounds, characterized by

a) for the manufacture of carboxamides of the general formula

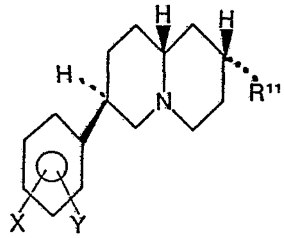

II

wherein X and Y signify the same as in claim 1 and $R^{11}$ represents acylamino, which can be further substituted on the nitrogen atom by lower-alkyl or phenyl optionally substituted by halogen, lower-alkyl or lower-alkoxy,
acylating a primary or secondary amine of the general formula

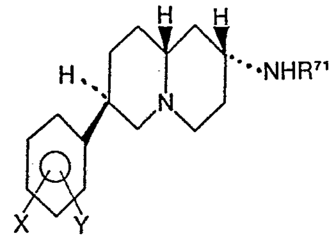

V

wherein X and Y signify the same as in claim 1 and $R^{71}$ represents hydrogen, lower-alkyl or phenyl, which can be substituted by halogen, lower-alkyl or lower-alkoxy, or

b) for the manufacture of ketones of the general formula

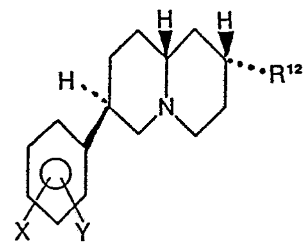

III

in which X and Y signify the same as in claim 1 and $R^{12}$ represents acyl,
treating a compound of the general formula

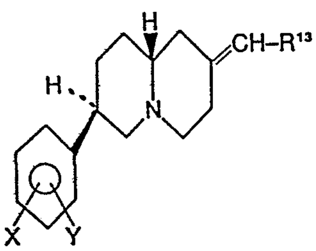

VI

in which X and Y signify the same as in claim 1 and the ylidene group $=CH-R^{13}$ corresponds to the acyl residue $R^{12}$ in which the oxo function has been replaced by hydrogen,
with diborane and hydrogen peroxide and subjecting the carbinol obtained to an oxidation; or

c) for the manufacture of a compound of the general formula

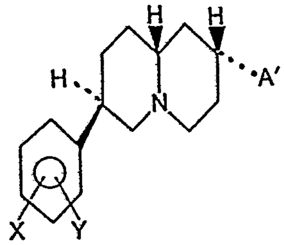

IV–3

wherein X and Y signify the same as in claim 1 and A′ is as A, whereby, however, $R^2$ represents oxygen and $R^4$ and $R^5$ are different from bromine and iodine,
catalytically hydrogenating a compound of the general formula

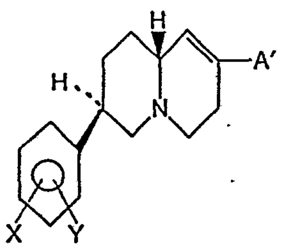

VII

wherein X, Y and A′ signify the same as in claim 1, or

d) for the manufacture of a compound of the general formula

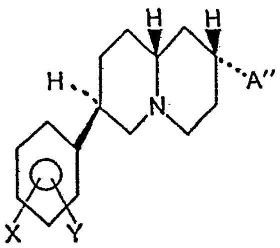

IV–4

wherein X and Y signify the same as in claim 1 and A″ is as A, whereby, however, $R^2$ represents oxygen and $R^3$ represents hydrogen,
condensing a phenylene-diamine of the general formula

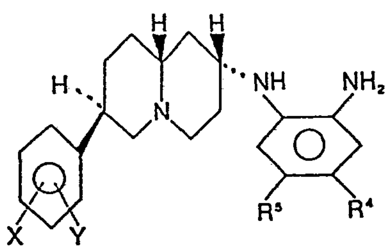

VIII

wherein X, Y, $R^4$ and $R^5$ signify the same as in claim 1,
with a cyclizing agent of the general formula

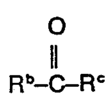

IX

wherein $R^b$ and $R^c$ signify halogen, amino or 1-imidazolyl,
or with acetic acid or an orthoacetic acid ester, or

e) for the manufacture of compounds of the general formula

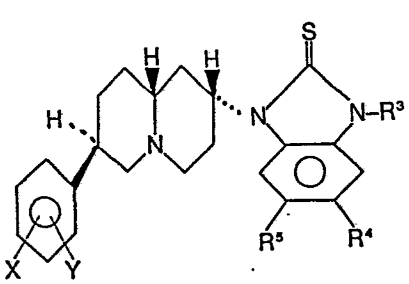

IV–5

wherein X, Y as well as $R^3$–$R^5$ signify the same as in claim 1,
either reacting a corresponding oxo compound of the general formula

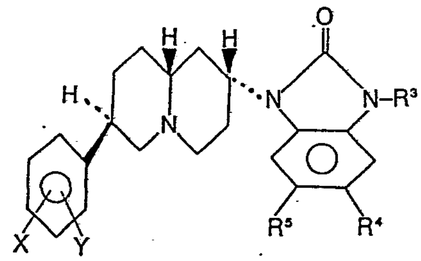

IV–6

wherein X, Y as well as $R^3$–$R^5$ signify the same as in claim 1,
with $P_2S_5$, or reacting a phenylene-diamine of general formula VIII above with thiophosgene, thiocarbonyldiimidazole or $CS_2$ and, if desired, subsequently N-alkylating the product, or

f) for the manufacture of compounds of the general formula

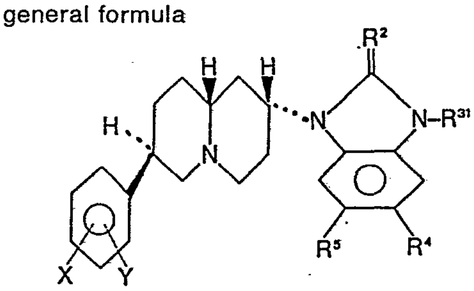

IV–7

wherein X, Y, $R^2$, $R^4$ and $R^5$ signify the same as in claim 1 and $R^{31}$ represents lower-alkyl,
N-alkylating a compound of the general formula

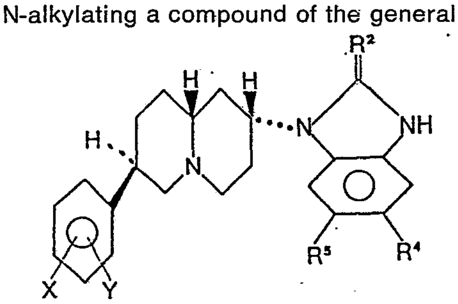

IV–8

wherein X, Y, $R^2$, $R^4$ and $R^5$ signify the same as in claim 1,
whereafter racemates obtained are resolved if desired and bases obtained are converted into acid addition salts if desired.

33. Medicaments, especially for use as antipsychotics and/or analgesics, containing a phenylquinolizidine of formula I given previously in the form of the racemate or of the enantiomers or an acid addition salt of such a compound.

**Claims for the Contracting state: AT**

1. A process for the manufacture of phenyl-quinolizidines of the general formula

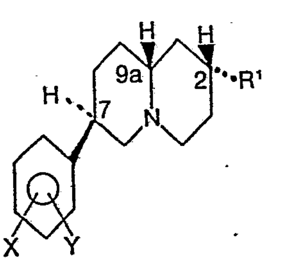

I

wherein X, Y and $R^1$ signify the following:

X: hydrogen, fluorine, chlorine, lower-alkoxy, lower-alkyl or trifluoromethyl,

Y: hydrogen, fluorine, chlorine, lower-alkoxy or lower-alkyl,

$R^1$: acyl, acylamino, which can be further substituted on the nitrogen atom by lower-alkyl or phenyl optionally substituted by halogen, lower-alkyl or lower-alkoxy, or a group A with the significance

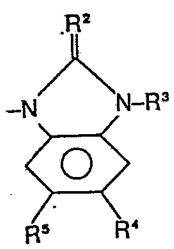

or

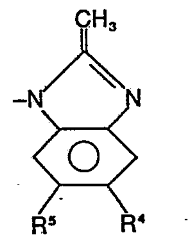

A

with the following significance for $R^2$–$R^5$:

$R^2$: oxygen or sulphur,

$R^3$: hydrogen or lower-alkyl,

$R^4$ and $R^5$: one is hydrogen, the other is bromine, iodine, cyano, lower-alkoxycarbonyl or sulphamoyl, whereby the groups denoted as lower have 1–6 carbon atoms and the residues denoted as acyl signify lower-alkanoyl, $C_4$–$C_7$-cycloalkylcarbonyl, benzoyl, which is optionally substituted by halogen, amino, lower-alkoxy, sulphamoyl and/or lower-alkylsulphonyl, 2-thiophenecarbonyl, 2-furancarbonyl or 2-oxo-1-pyrrolidinecarbonyl and the residues denoted as acylamino signify lower-alkanoylamino, $C_4$–$C_7$-cycloalkylcarbonylamino, 2-thiophenecarbonylamino, 2-furancarbonylamino or 2-oxo-1-pyrrolidinecarbonylamino,

in the form of the racemate or of the enantiomers, as well as of acid addition salts of such compounds, characterized by

a) for the manufacture of carboxamides of the general formula

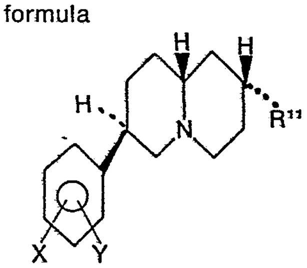

II

wherein X and Y signify the same as above and $R^{11}$ represents acylamino, which can be further substituted on the nitrogen atom by lower-alkyl or phenyl optionally substituted by halogen, lower-alkyl or lower-alkoxy,

acylating a primary or secondary amine of the general formula

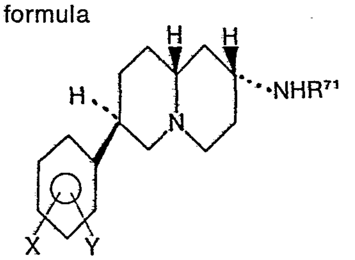

V

wherein X and Y signify the same as above and $R^{71}$ represents hydrogen, lower-alkyl or phenyl, which can be substituted by halogen, lower-alkyl or lower-alkoxy, or

b) for the manufacture of ketones of the general formula

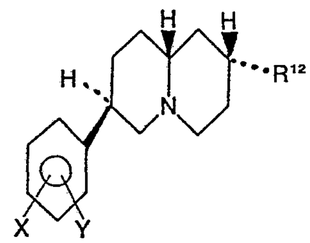

III

in which X and Y signify the same as above and $R^{12}$ represents acyl,

treating a compound of the general formula

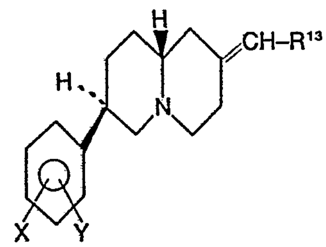

VI

in which X and Y signify the same as above and the ylidene group $=CH-R^{13}$ corresponds to the acyl residue $R^{12}$ in which the oxo function has been replaced by hydrogen,

with diborane and hydrogen peroxide and subjecting the carbinol obtained to an oxidation; or

c) for the manufacture of a compound of the general formula

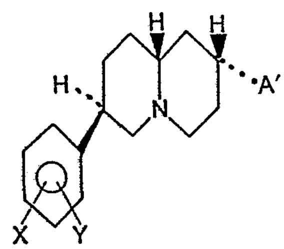

IV–3

wherein X and Y signify the same as above and A′ is as A, whereby, however, $R^2$ represents oxygen and $R^4$ and $R^5$ are different from bromine and iodine,

catalytically hydrogenating a compound of the general formula

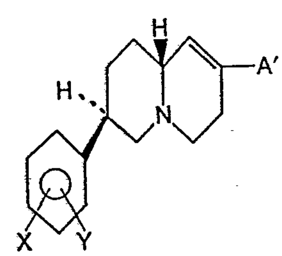

VII

wherein X, Y and A′ signify the same as above, or

d) for the manufacture of a compound of the general formula

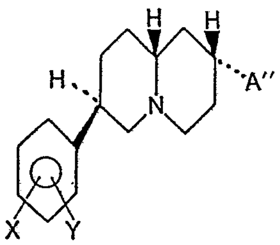

IV-4

wherein X and Y signify the same as above and A″ is as A, whereby, however, $R^2$ represents oxygen and $R^3$ represents hydrogen,
condensing a phenylene-diamine of the general formula

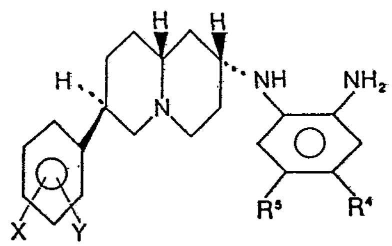

VIII

wherein X, Y, $R^4$ and $R^5$ signify the same as above, with a cyclizing agent of the general formula

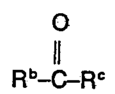

IX

wherein $R^b$ and $R^c$ signify halogen, amino or 1-imidazolyl,
or with acetic acid or an orthoacetic acid ester, or

e) for the manufacture of compounds of the general formula

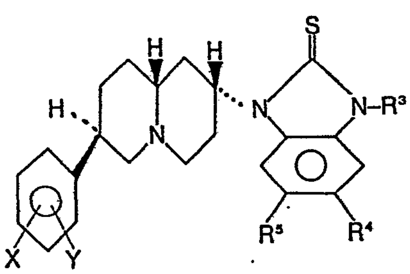

IV-5

wherein X, Y as well as $R^3$–$R^5$ signify the same as above,
either reacting a corresponding oxo compound of the general formula

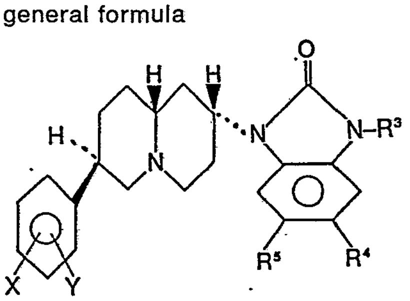

IV-6

wherein X, Y as well as $R^3$–$R^5$ signify the same as above,
with $P_2S_5$, or reacting a phenylene-diamine of general formula VIII above with thiophosgene, thiocarbonyldiimidazole or $CS_2$ and, if desired, subsequently N-alkylating the product, or

f) for the manufacture of compounds of the general formula

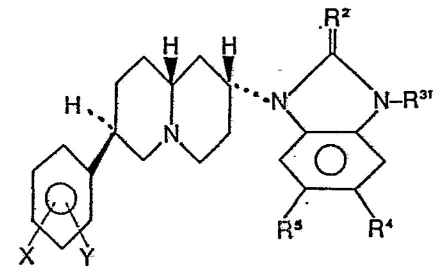

IV-7

wherein X, Y, $R^2$, $R^4$ and $R^5$ signify the same as above and $R^{31}$ represents lower-alkyl,
N-alkylating a compound of the general formula

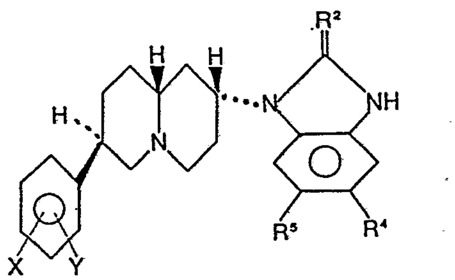

IV-8

wherein X, Y, $R^2$, $R^4$ and $R^5$ signify the same as above,
whereafter racemates obtained are resolved if desired and bases obtained are converted into acid addition salts if desired.

2. A process according to claim 1 for the manufacture of compounds of the general formula

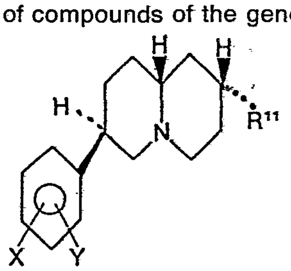

II

wherein X and Y signify the same as in claim 1 and $R^{11}$ represents acylamino, which can be further substituted on the nitrogen atom by lower-alkyl or phenyl optionally substituted by halogen, lower-alkyl or lower-alkoxy,
in the form of the racemate or of the enantiomers as well as of acid addition salts of such compounds, characterized in that correspondingly substituted starting compounds are used.

3. A process according to claim 2 for the manufacture of compounds of the general formula

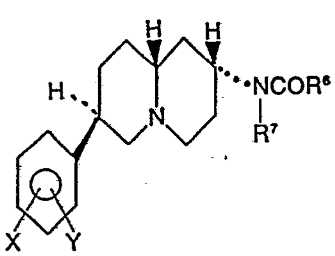

II-I

wherein X and Y signify the same as in claim 1, $R^6$ represents lower-alkyl and $R^7$ represents lower-

alkyl or phenyl, which can be substituted by halogen, lower-alkyl or lower-alkoxy,
in the form of the racemate or of the enantiomers as well as of acid addition salts of such compounds, characterized in that correspondingly substituted starting compounds are used.

4. A process according to claim 2 for the manufacture of compounds in which Y represents o-fluoro or o-chloro, X represents hydrogen and $R^{11}$ represents propionanilido, cyclopropanecarboxamido, 2-thiophenecarboxamido or 2-oxo-1-pyrrolidinecarboxamido, characterized in that correspondingly substituted starting compounds are used.

5. A process according to claim 2 for the manufacture of compounds in which Y represents o-fluoro or o-chloro, X represents hydrogen and $R^{11}$ represents 3′-chloro-propionanilido, 4′-chloropropionanilido or 4′-fluoro-propionanilido, characterized in that correspondingly substituted starting compounds are used.

6. A process according to claim 2 for the manufacture of compounds in which X and Y represent hydrogen and $R^{11}$ represents propionanilido or 4′-fluoropropionanilido.

7. A process according to claim 2 for the manufacture of
rac-N-[(9aβH)-7β-(o-chlorophenyl)octahydro-2H-quinolizin-2α-yl]cyclopropanecarboxamide
in the form of the racemate or of the enantiomers, as well as of acid addition salts of such compounds, characterized in that correspondingly substituted starting compounds are used.

8. A process according to claim 2 for the manufacture of
rac-N-[(9aβH)-7β-(o-chlorophenyl)octahydro-2H-quinolizin-2α-yl]-2-thiophenecarboxamide
in the form of the racemate or of the enantiomers, as well as of acid addition salts of such compounds, characterized in that correspondingly substituted starting compounds are used.

9. A process according to claim 2 for the manufacture of
rac-N-[(9aβH)-7β-(o-chlorophenyl)octahydro-2H-quinolizin-2α-yl]-2-oxo-1-pyrrolidinecarboxamide
in the form of the racemate or of the enantiomers, as well as of acid addition salts of such compounds, characterized in that correspondingly substituted starting compounds are used.

10. A process according to claim 2 for the manufacture of
rac-N-[(9aβH)-7β-(o-chlorophenyl)octahydro-2H-quinolizin-2α-yl]-propionanilide
in the form of the racemate or of the enantiomers, as well as acid addition salts of such compounds, characterized in that correspondingly substituted starting compounds are used.

11. A process according to claim 2 for the manufacture of
rac-4′-chloro-N-[(9aβH)-7β-(o-chlorophenyl)octahydro-2H-quinolizin-2α-yl]propionanilide
in the form of the racemate or of the enantiomers, as well as of acid addition salts of such compounds, characterized in that correspondingly substituted starting compounds are used.

12. A process according to claim 2 for the manufacture of
rac-3′-chloro-N-[(9aβH)-7β-(o-chlorophenyl)-octahydro-2H-quinolizin-2α-yl]-propionanilide
in the form of the racemate or of the enantiomers, as well as of acid addition salts of such compounds, characterized in that correspondingly substituted starting compounds are used.

13. A process according to claim 2 for the manufacture of
rac-N-[(9aβH)-7β-(o-fluorophenyl)-octahydro-2H-quinolizin-2α-yl]-propionanilide
in the form of the racemate or of the enantiomers, as well as of acid addition salts of such compounds, characterized in that correspondingly substituted starting compounds are used.

14. A process according to claim 2 for the manufacture of
rac-4′-fluoro-N-[(9aβH)-7β-(o-fluorophenyl)-octahydro-2H-quinolizin-2α-yl]-propionanilide
in the form of the racemate or of the enantiomers, as well as of acid addition salts of such compounds, characterized in that correspondingly substituted starting compounds are used.

15. A process according to claim 2 for the manufacture of
rac-4′-fluoro-N-[(9aβH)-7β-phenyl-octahydro-2H-quinolizin-2α-yl)-propionanilide
in the form of the racemate or of the enantiomers, as well as of acid addition salts of such compounds, characterized in that correspondingly substituted starting compounds are used.

16. A process according to claim 2 for the manufacture of
rac-N-[(9aβH)-7β-(o-fluorophenyl)-octahydro-2H-quinolizin-2α-yl]-acetanilide
in the form of the racemate or of the enantiomers, as well as of acid addition salts of such compounds, characterized in that correspondingly substituted starting compounds are used.

17. A process according to claim 2 for the manufacture of
rac-N-[(9aβH)-7β-(o-chlorophenyl)-octahydro-2H-quinolizin-2α-yl]-N-butylpropionamide
in the form of the racemate or of the enantiomers, as well as of acid addition salts of such compounds, characterized in that correspondingly substituted starting compounds are used.

18. A process according to claim 2 for the manufacture of
rac-N-[(9aβH)-7β-phenyl-octahydro-2H-quinolizin-2α-yl]-propionanilide
in the form of the racemate or of the enantiomers, as well as of acid addition salts of such compounds, characterized in that correspondingly substituted starting compounds are used.

19. A process according to claim 1 for the manufacture of compounds of the general formula

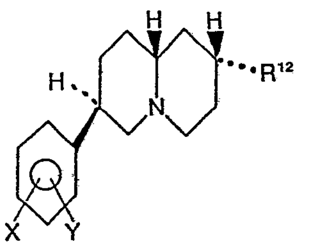

III

wherein X and Y signify the same as in claim 1 and $R^{12}$ represents acyl, in the form of the racemate or of the enantiomers as well as of acid addition salts of such compounds, characterized in that correspondingly substituted starting compounds are used.

20. A process according to claim 19 for the manufacture of compounds in which Y represents o-fluoro or o-chloro, X represents hydrogen and $R^{12}$ represents p-fluorobenzoyl, characterized in that correspondingly substituted starting compounds are used.

21. A process according to claim 20 for the manufacture of rac-(9aβH)-7β-(o-chlorophenyl)octahydro-2H-quinolizin-2α-yl] p-fluorophenylketone in the form of the racemate or of the enantiomers, as well as of acid addition salts of such compounds, characterized in that correspondingly substituted starting compounds are used.

22. A process according to claim 1 for the manufacture of compounds of the general formula

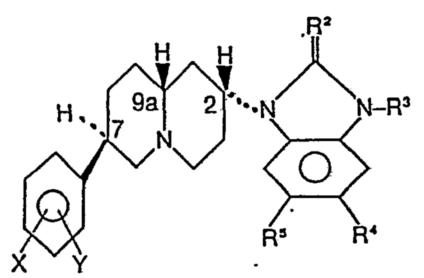

IV–1

wherein X, Y as well as $R^2$–$R^5$ signify the same as in claim 1, in the form of the racemate or of the enantiomers, as well as of acid addition salts of such compounds, characterized in that correspondingly substituted starting compounds are used.

23. A process according to claim 22 for the manufacture of rac-1-[(9aβH)-7β-(o-fluorophenyl)-octahydro-2H-quinolizin-2α-yl]-5-bromo-2-benzimidazolinone in the form of the racemate or of the enantiomers, as well as of acid addition salts of such compounds, characterized in that correspondingly substituted starting compounds are used.

24. A process according to claim 22 for the manufacture of rac-1-[(9aβH)-7β-(o-fluorophenyl)-octahydro-2H-quinolizin-2α-yl]-5-iodo-2-benzimidazolinone in the form of the racemate or of the enantiomers, as well as of acid addition salts of such compounds, characterized in that correspondingly substituted starting compounds are used.

25. A process according to claim 22 for the manufacture of rac-1-[(9aβH)-7β-(o-fluorophenyl)-octahydro-2H-quinolizin-2α-yl]-5-cyano-2-benzimidazolinone in the form of the racemate or of the enantiomers, as well as of acid addition salts of such compounds, characterized in that correspondingly substituted starting compounds are used.

26. A process according to claim 1 for the manufacture of compounds of the general formula

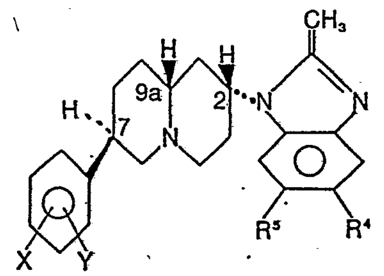

IV–2

wherein X, Y, $R^4$ and $R^5$ signify the same as in claim 1, in the form of the racemate or of the enantiomers, as well as of acid addition salts of such compounds, characterized in that correspondingly substituted starting compounds are used.

**Revendications pour les Etats contractants: BE, CH/Li, DE, FR, GB, IT, LU, NL, SE**

1. Phénylquinolizidines de formule générale

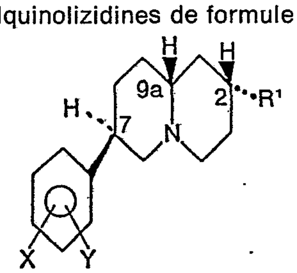

I

dans laquelle X, Y et $R^1$ ont les significations suivantes:

X: l'hydrogène, le fluor, le chlore, un groupe alcoxy inférieur, alkyle inférieur ou trifluorométhyle,

Y: l'hydrogène, le fluor, le chlore, un groupe alcoxy inférieur ou alkyle inférieur,

$R^1$: un groupe acyle, acylamino qui peut encore être substitué sur l'atome d'azote par un groupe alkyle inférieur ou par un groupe phényle éventuellement substitué par des halogènes, des groupes alkyle inférieurs ou alcoxy inférieurs, ou un groupe A de formule

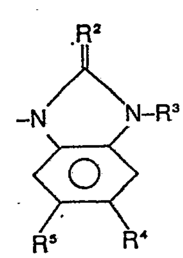

ou

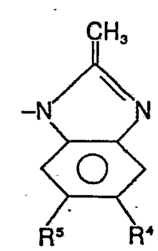

A

dans lesquelles $R^2$ à $R^5$ ont les significations suivantes:

$R^2$: l'oxygène ou le soufre,

$R^3$: l'hydrogène ou un groupe alkyle inférieur,

$R^4$ et $R^5$: l'un des symboles représente l'hydrogène et l'autre le brome, l'iode, un groupe cyano, alcoxy-carbonyle inférieur ou sulfamoyle,

à l'état du racémate ou des énantiomères, ainsi que les sels de ces composés formés par addition avec des acides, les groupes appelés «inférieurs» contenant 1 à 6 atomes de carbone et les groupes acyle étant des groupes alcanoyle inférieurs, cycloalkylcarbonyle en C 4–C 7, benzoyle éventuellement substitué par des halogènes, des groupes amino, alcoxy inférieurs, sulfamoyle et/ou alkylsulfonyle inférieurs, des groupes 2-thiophène-carbonyle, 2-furanne-carbonyle ou 2-oxo-1-pyrrolidine-carbonyle, et les groupes acylamino représentant des groupes alkanoyl(inférieur)amino, $C_4$–$C_7$-cycloalkylcarbonylamino, 2-thiophène-carbonylamino, 2-furanne-carbonylamino ou 2-oxo-1-pyrrolidine-carbonylamino.

2. Composés selon la revendication 1, de formule générale

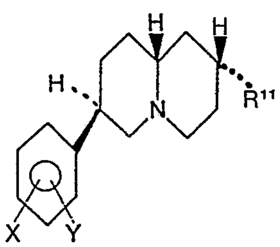

II

dans laquelle X et Y sont les significations indiquées dans la revendication 1 et $R^{11}$ représente un groupe acylamino qui peut encore être substitué sur l'atome d'azote par un groupe alkyle inférieur ou par un groupe phényle éventuellement substitué par des halogènes, des groupes alkyle inférieurs ou alcoxy inférieurs,

à l'état du racémate ou des énantiomères, ainsi que les sels de ces composés formés par addition avec des acides.

3. Composés selon la revendication 2, de formule générale

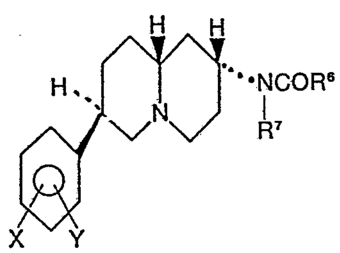

II–I

dans laquelle X et Y ont les significations indiquées dans la revendication 1, $R^6$ représente un groupe alkyle inférieur et $R^7$ représente un groupe alkyle inférieur ou phényle qui peut être substitué par des halogènes, des groupes alkyle inférieurs ou alcoxy inférieurs,

à l'état du racémate ou des énantiomères, ainsi que les sels de ces composés formés par addition avec des acides.

4. Composés selon la revendication 2, dans lesquels Y représente un substituant o-fluoro ou o-chloro, X représente l'hydrogène et $R^{11}$ représente un groupe propionanilido, cyclopropane-carboxamido, 2-thiophène-carboxamido ou 2-oxo-1-pyrrolidine-carboxamido.

5. Composés selon la revendication 2, dans lesquels Y représente un substituant o-fluoro ou o-chloro, X représente l'hydrogène et $R^{11}$ un groupe 3′-chloropropionanilido, 4′-chloropropionanilido ou 4′-fluoropropionanilido.

6. Composés selon la revendication 2, dans lesquels X et Y représentent l'hydrogène et $R^{11}$ un groupe propionanilido ou 4′-fluoropropionanilido.

7. Le rac-N-[(9a bêtaH)-7 bêta-(o-chlorophényl)-octahydro-2H-quinolizine-2-alpha-yl]-cyclopropane-carboxamide

à l'état du racémate ou des énantiomères, ainsi que les sels de ces composés formès par addition avec des acides.

8. Le rac-N-[(9a bêtaH)-7 bêta-(o-chlorophényl)-octahydro-2H-quinolizine-2-alpha-yl]-2-thiophène-carboxamide

à l'état du racémate ou des énantiomères, ainsi que les sels de ces composés formés par addition avec des acides.

9. Le rac-N-[(9a bêtaH)-7 bêta-(o-chlorophényl)-octahydro-2H-quinolizine-2-alpha-yl]-2-oxo-1-pyrrolidine-carboxamide

à l'état du racémate ou des énantiomères, ainsi que les sels de ces composés formés par addition avec des acides.

10. Le rac-N-[(9a bêtaH)-7 bêta-(o-chlorophényl)-octahydro-2H-quinolizine-2-alpha-yl]-propionanilide

à l'état du racémate ou des énantiomères, ainsi que les sels de ces composés formés par addition avec des acides.

11. Le rac-4′-chloro-N-[(9a bêtaH)-7-bêta-(o-chlorophényl)-octahydro-2H-quinolizine-2 alpha-yl]-propionanilide

à l'état du racémate ou des énantiomères, ainsi que les sels de ces composés formés par addition avec des acides.

12. Le rac-3′-chloro-N-[(9a bêtaH)-7 bêta-(o-chlorophényl)-octahydro-2H-quinolizine-2 alpha-yl]-propionanilide

à l'état du racémate ou des énantiomères, ainsi que les sels de ces composés formés par addition avec des acides.

13. Le rac-N-(9a bêtaH)-7 bêta-(o-fluorophényl)-octahydro-2H-quinolizine-2 alpha-yl)-propionanilide

á l'état du racémate ou des énantiomères, ainsi que les sels de ces composés formés par addition avec des acides.

14. Le rac-4′-fluoro-N-[(9a bêtaH)-7 bêta-(o-fluorophényl)-octahydro-2H-quinolizine-2 alpha-yl]-propionanilide

à l'état du racémate ou des énantiomères, ainsi que les sels de ces composés formés par addition avec des acides.

15. Le rac-4′-fluoro-N-[(9a bêtaH)-7 bêtaphényl-octahydro-2H-quinolizine-2 alpha-yl]-propionanilide

à l'état du racémate ou des énantiomères, ainsi

que les sels de ces composés formés par addition avec des acides.

16. Le rac-N-[(9a bêtaH)-7 bêta-(o-fluorophényl)-octahydro-2H-quinolizine-2 alpha-yl]-acétanilide

à l'état du racémate ou des énantiomères, ainsi que les sels de ces composés formés par addition avec des acides.

17. Le rac-N-[(9a bêtaH)-7 bêta-(o-chlorophényl)-octahydro-2H-quinolizine-2 alpha-yl]-N-butylpropionamide

à l'état du racémate ou des énantiomères, ainsi que les sels de ces composés formés par addition avec des acides.

18. Le rac-N-[(9a bêtaH)-7 bêta-phényloctahydro-2H-quinolizine-2 alpha-yl]-propionanilide

à l'état du racémate ou des énantiomères, ainsi que les sels de ces composés formés par addition avec des acides.

19. Composés selon la revendication 1, de formule générale

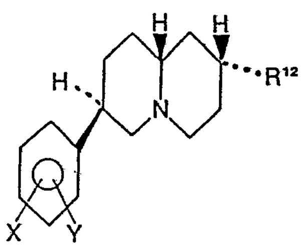

III

dans laquelle X et Y ont les significations indiquées dans la revendication 1 et $R^{12}$ représente un groupe acyle,

à l'état du racémate ou des énantiomères, ainsi que les sels de ces composés formés par addition avec des acides.

20. Composés selon la revendication 19, dans lesquels Y représente un substituant o-fluoro ou o-chloro, X représente l'hydrogène et $R^{12}$ un groupe p-fluorobenzoyle.

21. La rac-(9a bêtaH)-7 bêta-(o-chlorophényl)-octahydro-2H-quinolizine-2 alpha-yl)-p-fluorophénylcétone

à l'état du racémate ou des énantiomères, ainsi que les sels de ces composés formés par addition avec des acides.

22. Composés selon la revendication 1, de formule générale

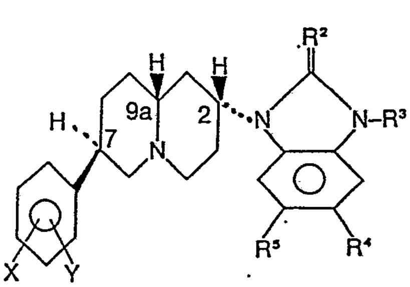

IV–1

dans laquelle X, Y et $R^2$ à $R^5$ ont les significations indiquées dans la revendication 1, à l'état du racémate ou des énantiomères, ainsi que les sels de ces composés formés par addition avec des acides.

23. La rac-1-[(9a bêtaH)-7 bêta-(o-fluorophényl)-octahydro-2H-quinolizine-2 alpha-yl]-5-bromo-2-benzimidazolinone

à l'état de racémate ou des énantiomères, ainsi que les sels de ces composés formés par addition avec des acides.

24. La rac-1-[(9a bêtaH)-7 bêta-(o-fluorophényl)-octahydro-2H-quinolizine-2 alpha-yl]-5-iodo-2-benzimidazolinone

à l'état de racémate ou des énantiomères, ainsi que les sels de ces composés formés par addition avec des acides.

25. La rac-1-[(9a bêtaH)-7 bêta-(o-fluorophényl)-octahydro-2H-quinolizine-2 alpha-yl]-5-cyano-2-benzimidazolinone

à l'état du racémate ou des énantiomères, ainsi que les sels de ces composés formés par addition avec des acides.

26. Composés selon la revendication 1, de formule générale

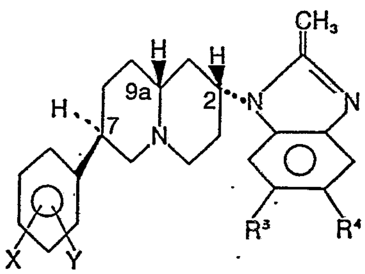

IV–2

dans laquelle X, Y, $R^4$ et $R^5$ ont les significations indiquées dans la revendication 1, à l'état du racémate ou des énantiomères, ainsi que les sels de ces composés formés par addition avec des acides.

27. Phénylquinolizidines insaturées de formule générale

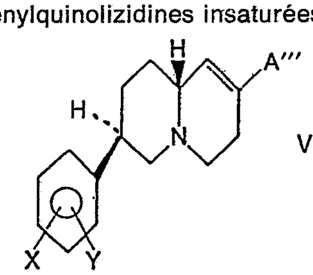

VII+XIII

dans laquelle X et Y ont les significations indiquées dans la revendication 1, et A''' a les significations de A dans la revendication 1, étant précisé que lorsque $R^3$ représente un groupe alkyle inférieur, $R^2$ représente l'oxygène, à l'état du racémate et des énantiomères.

28. Phénylène-diamines de formule générale

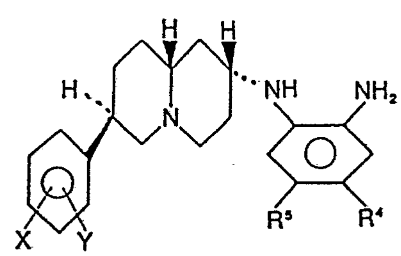

VIII

dans laquelle X, Y, $R^4$ et $R^5$ ont les significations indiquées dans la revendication 1, à l'état du racémate et des énantiomères.

29. Amines primaires ou secondaires de formule générale

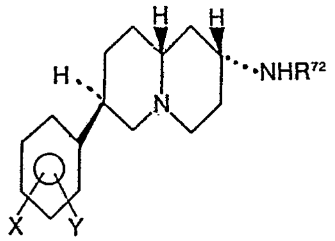

V–1

dans laquelle X et Y ont les significations indiquées dans la revendication 1, et $R^{72}$ représente un groupe alkyle inférieur ou phényle qui peut être substitué par des halogènes, des groupes alkyle inférieurs ou alcoxy inférieurs, à l'état du racémate et des énantiomères.

30. Composés en ylidène, de formule générale

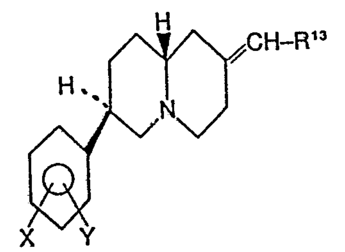

VI

dans laquelle X et Y ont les significations indiquées dans la revendication 1, et le groupe en ylidène $=CH-R^{13}$ correspond au groupe acyle $R^{12}$ de la revendication 19 dans lequel la fonction oxo a été remplacée par l'hydrogène, à l'état du racémate et des énantiomères.

31. Phénylquinolizidines selon l'une des revendications 1 à 17, pour l'utilisation dans un procédé thérapeutique en tant que substances actives pharmacologiques, en particulier en tant que substances actives anti-psychotiques et/ou analgésiques.

32. Procédé de préparation des phényl-quinolizidines de formule générale I de la revendication 1, à l'état du racémate ou des énantiomères, ainsi que des sels de ces composés formés par addition avec des acides, caractérisé en ce que

a) pour la préparation des carboxamides de formule générale

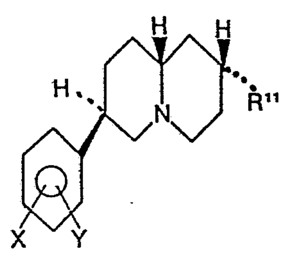

II

dans laquelle X et Y ont les significations indiquées dans la revendication 1 et $R^{11}$ représente un groupe acylamino qui peut encore être substitué sur l'atome d'azote par un groupe alkyle inférieur ou par un groupe phényle lui-même éventuellement substitué par des halogènes, des groupes alkyle inférieurs ou alcoxy inférieurs, on acyle une amine primaire ou secondaire de formule générale

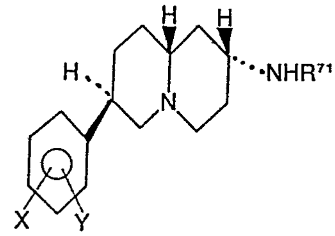

V

dans laquelle X et Y ont les significations indiquées dans la revendication 1 et $R^{71}$ représente l'hydrogène, un groupe alkyle inférieur ou phényle qui peut être substitué par des halogènes, des groupes alkyle inférieurs ou alcoxy inférieurs, ou bien

b) pour la préparation des cétones de formule générale

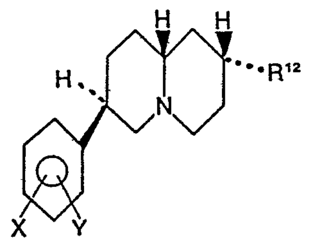

III

dans laquelle X et Y ont les significations indiquées dans la revendication 1 et $R^{12}$ représente un groupe acyle, on traite un composé de formule générale

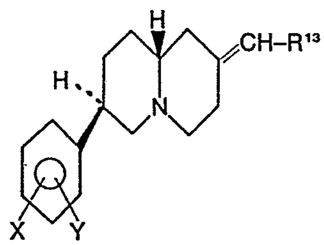

VI

dans laquelle X et Y ont les significations indiquées dans la revendication 1 et le groupe en ylidène $=CH-R^{13}$ correspond au groupe acyle $R^{12}$ dans lequel la fonction oxo a été remplacée par l'hydrogène, par le diborane et le peroxyde d'hydrogène, et on soumet le carbinol obtenu à une oxydation; ou bien

c) pour la préparation d'un composé de formule générale

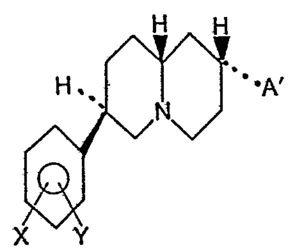

IV–3

dans laquelle X et Y ont les significations indiquées dans la revendication 1, et A′ a la même signification que A, $R^2$ représentant toutefois

l'oxygène et $R^4$ et $R^5$ des substituants autres que le brome et l'iode, on soumet un composé de formule générale

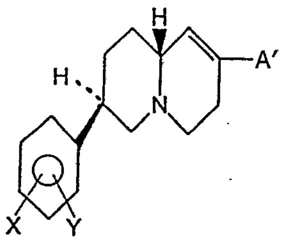

VII

dans laquelle X, Y et A' ont les significations indiquées dans la revendication 1, à hydrogénation catalytique, ou bien

d) pour préparer un composé de formule générale

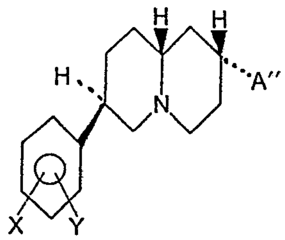

IV–4

dans laquelle X et Y ont les significations indiquées dans la revendication 1 et A'' a la même signification que A, $R^2$ représentant toutefois l'oxygène et $R^3$ l'hydrogène, on condense une phénylène-diamine de formule générale

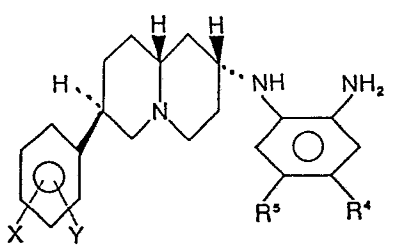

VIII

dans laquelle X, Y, $R^4$ et $R^5$ ont les significations indiquées dans la revendication 1, avec un agent cyclisant de formule générale

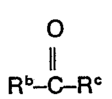

IX

dans laquelle $R^b$ et $R^c$ représentent des halogènes, des groupes amino ou 1-imidazolyle, ou avec l'acide acétique ou un ester orthoacétique, ou bien

e) pour préparer les composés de formule générale

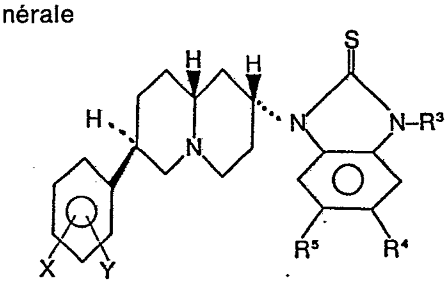

IV–5

dans laquelle X, Y et $R^3$ à $R^5$ ont les significations indiquées dans la revendication 1, ou bien on fait réagir un composé correspondant en oxo de formule générale

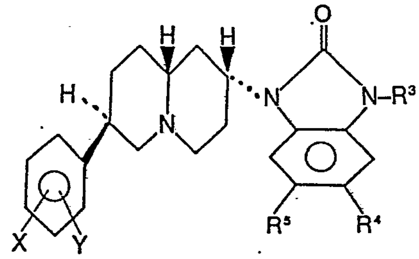

IV–6

dans laquelle X, Y et $R^3$ à $R^5$ ont les significations indiquées dans la revendication 1, avec $P_2S_5$, ou bien on fait réagir une phénylène-diamine de formule générale VIII ci-dessus avec le thiophosgène, le thiocarbonyl-diimidazole ou $CS_2$ et on soumet ensuite le cas échéant à une N-alkylation, ou bien

f) pour préparer les composés de formule générale

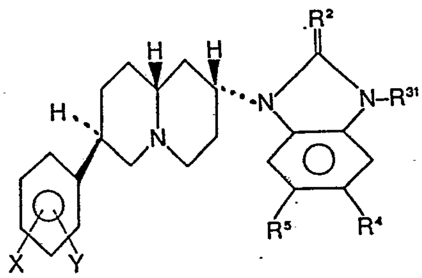

IV–7

dans laquelle X, Y, $R^2$, $R^4$ et $R^5$ ont les significations indiquées dans la revendication 1, et $R^{31}$ représente un groupe alkyle inférieur,
on soumet un composé de formule générale

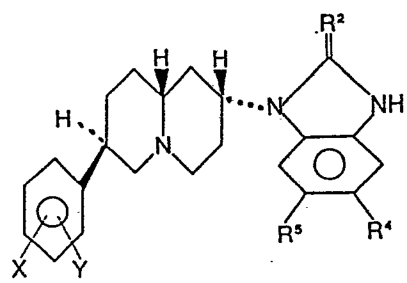

IV–8

dans laquelle X, Y, $R^2$, $R^4$ et $R^5$ ont les significations indiquées dans la revendication 1, à une N-alkylation, après quoi, le cas échéant, on soumet les racémates obtenus à resolution et on convertit le cas échéant les bases obtenues en sels par addition avec des acides.

33. Médicaments utilisables en particulier en tant qu'antipsychotiques et/ou analgésiques, contenant une phénylquinolizidine de formule I ci-dessus à l'état du racémate ou d'un énantiomère ou d'un sel d'un tel composé formé par addition avec un acide.

**Revendications pour l'Etat contractant: AT**

1. Procédé de préparation de phénylquinolizidines de formule générale

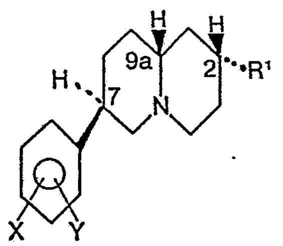

I

dans laquelle X, Y et $R^1$ ont les significations suivantes:

X: l'hydrogène, le fluor, le chlore, un groupe alcoxy inférieur, alkyle inférieur ou trifluorométhyle,

Y: l'hydrogène, le fluor, le chlore, un groupe alcoxy inférieur ou alkyle inférieur,

$R^1$: un groupe acyle, acylamino qui peut encore être substitué sur l'atome d'azote par un groupe alkyle inférieur ou par un groupe phényle éventuellement substitué par des halogènes, des groupes alkyle inférieurs ou alcoxy inférieurs, ou un groupe A de formule

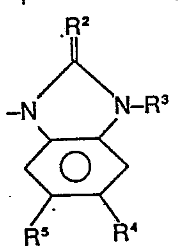

ou

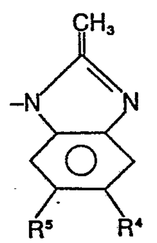

A

dans lesquelles $R^2$ à $R^5$ ont les significations suivantes:

$R^2$: l'oxygène ou le soufre,

$R^3$: l'hydrogène ou un groupe alkyle inférieur,

$R^4$ et $R^5$: l'un des symboles représente l'hydrogène et l'autre le brome, l'iode, un groupe cyano, alcoxycarbonyle inférieur ou sulfamoyle, les groupes appelés «inférieurs» contenant de 1 à 6 atomes de carbone et les groupes acyle étant des groupes alcanoyle inférieurs, cycloalkylcarbonyle en C 4–C 7, benzoyle éventuellement substitué par des halogènes, des groupes amino, alcoxy inférieurs, sulfamoyle et/ou alkylsulfonyle inférieurs, des groupes 2-thiophène-carbonyle, 2-furanne-carbonyle ou 2-oxo-1-pyrrolidine-carbonyle, et les groupes acylamino représentant des groupes alkanoyl(inférieur)amino, $C_4$–$C_7$-cycloalkylcarbonylamino, 2-thiophène-carbonylamino, 2-furanne-carbonylamino ou 2-oxo-1-pyrrolidine-carbonylamino à l'état du racémate ou des énantiomères, ainsi que des sels de ces composés formés par addition avec des acides, caractérisé en ce que:

a) pour la préparation des carboxamides de formule générale

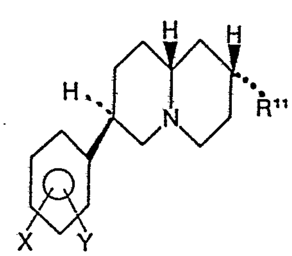

II

dans laquelle X et Y ont les significations indiquées ci-dessus et $R^{11}$ représente un groupe acylamino qui peut encore être substitué sur l'atome d'azote par un groupe alkyle inférieur ou par un groupe phényle lui-même éventuellement substitué par des halogènes, des groupes alkyle inférieurs ou alcoxy inférieurs, on acyle une amine primaire ou secondaire de formule générale

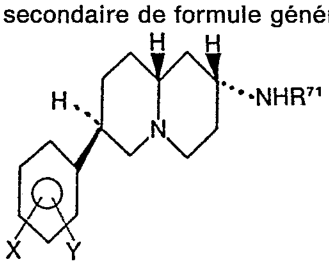

V

dans laquelle X et Y ont les significations indiquées ci-dessus et $R^{71}$ représente l'hydrogène, un groupe alkyle inférieur ou phényle qui peut être substitué par des halogènes, des groupes alkyle inférieurs ou alcoxy inférieurs, ou bien

b) pour la préparation des cétones de formule générale

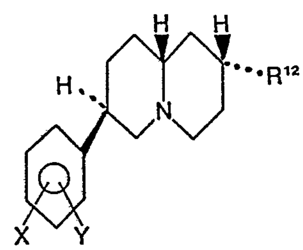

III

dans laquelle X et Y ont les significations indiquées ci-dessus et $R^{12}$ représente un groupe acyle, on traite un composé de formule générale

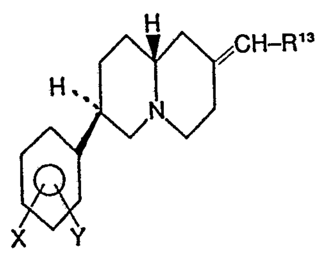

VI

dans laquelle X et Y ont les significations indiquées ci-dessus et le groupe en ylidène $=CH-R^{13}$ correspond au groupe acyle $R^{12}$ dans lequel la fonction oxo a été remplacée par l'hydrogène, par le diborane et le peroxyde d'hydrogène, et on soumet le carbinol obtenu à une oxydation; ou bien

c) pour la préparation d'un composé de formule générale

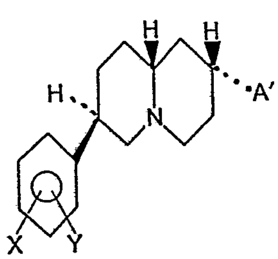

IV–3

dans laquelle X et Y ont les significations indiquées ci-dessus et A′ a la même signification que A, $R^2$ représentant toutefois l'oxygène et $R^4$ et $R^5$ des substituants autres que le brome et l'iode, on soumet un composé de formule générale

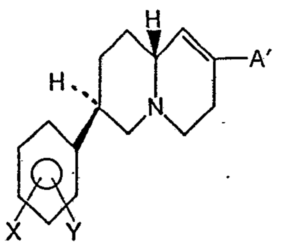

VII

dans laquelle X, Y et A′ ont les significations indiquées ci-dessus, à hydrogénation catalytique, ou bien

d) pour préparer un composé de formule générale

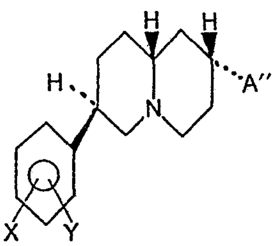

IV–4

dans laquelle X et Y ont les significations indiquées ci-dessus et A″ a la même signification que A, $R^2$ représente toutefois l'oxygène et $R^3$ l'hydrogène,
on condense une phénylène-diamine de formule générale

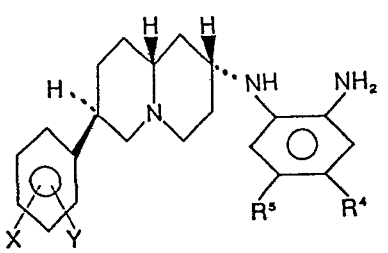

VIII

dans laquelle X, Y, $R^4$ et $R^5$ ont les significations indiquées ci-dessus, avec un agent cyclisant de formule générale

$$R^b{-}\overset{\overset{\displaystyle O}{\|}}{C}{-}R^c \qquad \text{IX}$$

dans laquelle $R^b$ et $R^c$ représentent des halogènes, des groupes amino ou 1-imidazolyle,
ou avec l'acide acétique ou un ester orthoacétique, ou bien

e) pour préparer les composés de formule générale

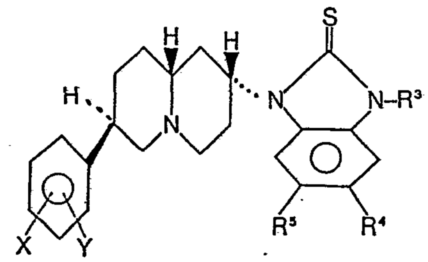

IV–5

dans laquelle X, Y et $R^3$ à $R^5$ ont les significations indiquées ci-dessus, ou bien on fait réagir un composé correspondant en oxo de formule générale

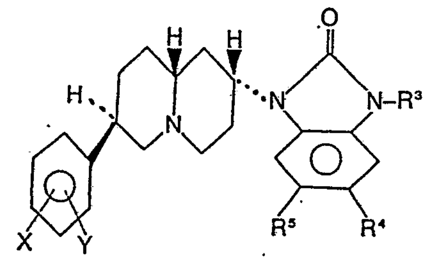

IV–6

dans laquelle X, Y et $R^3$ à $R^5$ ont les significations indiquées ci-dessus, avec $P_2S_5$, ou bien on fait réagir une phénylène-diamine de formule générale VIII ci-dessus avec le thiophosgène, le thiocarbonyldiimidazole ou $CS_2$ et on soumet ensuite le cas échéant à une N-alkylation, ou bien

f) pour préparer les composés de formule générale

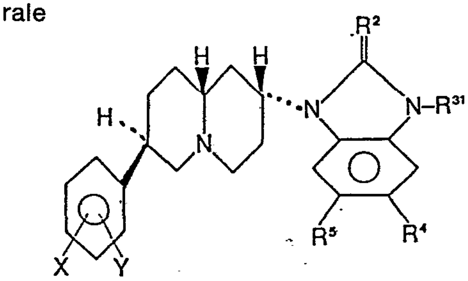

IV–7

dans laquelle X, Y, $R^2$, $R^4$ et $R^5$ ont les significations indiquées ci-dessus, et $R^{31}$ représente un groupe alkyle inférieur,
on soumet un composé de formule générale

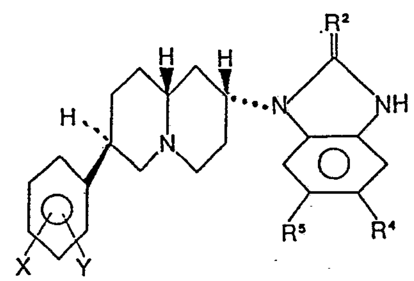

IV–8

dans laquelle X, Y, $R^2$, $R^4$ et $R^5$ ont les significations indiquées ci-dessus, à une N-alkylation, après quoi, le cas échéant, on soumet les racémates obtenus à résolution et on convertit le cas échéant les bases obtenues en sels par addition avec des acides.

2. Procédé selon la revendication 1, pour la préparation de composés de formule générale

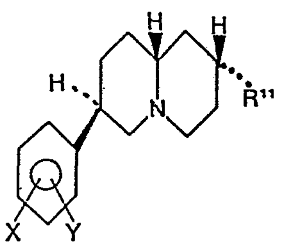

II

dans laquelle X et Y ont les significations indiquées dans la revendication 1, et $R^{11}$ représente un groupe acylamino qui peut encore être substitué sur l'atome d'azote par un groupe alkyle inférieur ou par un groupe phényle lui-même éventuellement substitué par des halogènes, des groupes alkyle inférieurs ou alcoxy inférieurs, à l'état du racémate ou des énantiomères, ainsi que des sels de ces composés formés par addition avec des acides, caractérisé en ce que l'on met en œuvre des composés de départ portant les substituants correspondants.

3. Procédé selon la revendication 2, pour la préparation de composés de formule générale

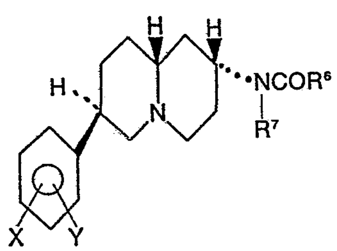

II-I

dans laquelle X et Y ont les significations indiquées dans la revendication 1, $R^6$ représente un groupe alkyle inférieur et $R^7$ un groupe alkyle inférieur ou un groupe phényle qui peut être substitué par des halogènes, des groupes alkyle inférieurs ou alcoxy inférieurs, à l'état du racémate ou des énantiomères, ainsi que des sels de ces composés formés par addition avec des acides, caractérisé en ce que l'on met en œuvre des composés de départ portant les substituants correspondants.

4. Procédé selon la revendication 2, pour la préparation de composés dans lesquels Y représente un substituant o-fluoro ou o-chloro, X représente l'hydrogène et $R^{11}$ un groupe propionanilido, cyclopropane-carboxamido, 2-thiophène-carboxamido, ou 2-oxo-1-pyrrolidine-carboxamido, caractérisé en ce que l'on met en œuvre des composés de départ portant les substituants correspondants.

5. Procédé selon la revendication 2, pour la préparation de composés dans lesquels Y représente un substituant o-fluoro ou o-chloro, X représente l'hydrogène et $R^{11}$ un groupe 3'-chloropropionanilido, 4'-chloropropionanilido ou 4'-fluoropropionanilido, caractérisé en ce que l'on met en œuvre des composés de départ portant les substituants correspondants.

6. Procédé selon la revendication 2, pour la préparation de composés dans lesquels X et Y représentent l'hydrogène et $R^{11}$ un groupe propionanilido ou 4'-fluoropropionanilido.

7. Procédé selon la revendication 2, pour la préparation du
rac-N-[(9a bêtaH)-7 bêta-(o-chlorophényl)-octahydro-2H-quinolizine-2 alphayl]-cyclopropanecarboxamide,
à l'état du racémate ou des énantiomères, ainsi que des sels de ces composés formés par addition avec des acides, caractérisé en ce que l'on met en œuvre des composés de départ portant les substituants correspondants.

8. Procédé selon la revendication 2, pour la préparation du
rac-N-[(9a bêtaH)-7 bêta-(o-chlorophényl)-octahydro-2H-quinolizine-2 alpha-yl]-2-thiophènecarboxamide,
à l'état du racémate ou des énantiomères, ainsi que des sels de ces composés formés par addition avec des acides, caractérisé en ce que l'on met en œuvre des composés de départ portant les substituants correspondants.

9. Procédé selon la revendication 2, pour la préparation du
rac-N-[(9a bêtaH)-7 bêta-(o-chlorophényl)-octahydro-2H-quinolizine-2 alpha-yl]-2-oxopyrrolidinecarboxamide
à l'état du racémate ou des énantiomères, ainsi que des sels de ces composés formés par addition avec des acides, caractérisé en ce que l'on met en œuvre des composés de départ portant les substituants correspondants.

10. Procédé selon la revendication 2, pour la préparation du
rac-N-[9a bêtaH)-7 bêta-(o-chlorophényl)-octahydro-2H-quinolizine-2 alpha-yl]-propionanilide,
à l'état du racémate ou des énantiomères, ainsi que des sels de ces composés formés par addition avec des acides, caractérisé en ce que l'on met en œuvre des composés de départ portant les substituants correspondants.

11. Procédé selon la revendication 2, pour la préparation du
rac-4'-chloro-N-[(9a bêtaH)-7 bêta-(o-chlorophényl)-octahydro-2H-quinolizine-2 alpha-yl]-propionanilide,
à l'état du racémate et des énantiomères, ainsi que des sels de ces composés formés par addition avec des acides, caractérisé en ce que l'on met en œuvre des composés de départ portant les substituants correspondants.

12. Procédé selon la revendication 2, pour la préparation du
rac-3-chloro-N-[(9a bêtaH)-7 bêta-(o-chlorophényl)-octahydro-2H-quinolizine-2 alpha-yl]-propionanilide,
à l'état du racémate et des énantiomères, ainsi que des sels de ces composés formés par addition avec des acides, caractérisé en ce que l'on met en œuvre des composés de départ portant les substituants correspondants.

13. Procédé selon la revendication 2, pour la préparation du
rac-N-[(9a bêtaH)-7 bêta-(o-fluorophényl)-octahydro-2H-quinolizine-2 alpha-yl)-propionanilide,

à l'état du racémate ou des énantiomères, ainsi que des sels de ces composés formés par addition avec des acides, caractérisé en ce que l'on met en œuvre des composés de départ portant les substituants correspondants.

14. Procédé selon la revendication 2, pour la préparation du
rac-4'-fluoro-N-[(9a bêtaH)-7 bêta-(o-fluoro-phényl)-octahydro-2H-quinolizine-2 alpha-yl]-propionanilide,
à l'état du racémate ou des énantiomères, ainsi que des sels de ces composés formés par addition avec des acides, caractérisé en ce que l'on met en œuvre des composés de départ portant les substituants correspondants.

15. Procédé selon la revendication 2, pour la préparation du
rac-4'-fluoro-N-[(9a bêtaH)-7 bêta-phényloctahydro-2H-quinolizine-2 alpha-yl)-propionanilide,
à l'état du racémate ou des énantiomères, ainsi que des sels de ces composés formés par addition avec des acides, caractérisé en ce que l'on met en œuvre des composés de départ portant les substituants correspondants.

16. Procédé selon la revendication 2, pour la préparation du
rac-N-[(9a bêtaH)-7 bêta-(o-fluorophényl)-octahydro-2H-quinolizine-2 alpha-yl]-acétanilide,
à l'état du racémate ou des énantiomères, ainsi que des sels de ces composés formés par addition avec des acides, caractérisé en ce que l'on met en œuvre des composés de départ portant les substituants correspondants.

17. Procédé selon la revendication 2, pour la préparation du
rac-N-[(9a bêtaH)-7 bêta-(o-chlorophényl)-octahydro-2H-quinolizine-2 alpha-yl]-N-butyl-propionamide,
à l'état du racémate ou des énantiomères, ainsi que des sels de ces composés formés par addition avec des acides, caractérisé en ce que l'on met en œuvre des composés de départ portant les substituants correspondants.

18. Procédé selon la revendication 2, pour la préparation du
rac-N-[(9a bêtaH)-7 bêta-phényl-octahydro-2H-quinolizine-2 alpha-yl]-propionanilide,
à l'état du racémate ou des énantiomères, ainsi que des sels de ces composés formés par addition avec des acides, caractérisé en ce que l'on met en œuvre des composés de départ portant les substituants correspondants.

19. Procédé selon la revendication 1, pour la préparation de composés de formule générale

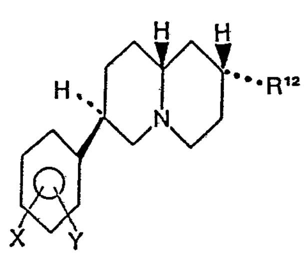

III

dans laquelle X et Y ont les significations indiquées dans la revendication 1, et $R^{12}$ représente un groupe acyle, à l'état du racémate ou des énantiomères, ainsi que des sels de ces composés formés par addition avec des acides, caractérisé en ce que l'on met en œuvre des composés de départ portant les substituants correspondants.

20. Procédé selon la revendication 19, pour la préparation de composés dans lesquels Y représente un substituant o-fluoro ou o-chloro, X l'hydrogène et $R^{12}$ un groupe p-fluorobenzoyle, caractérisé en ce que l'on met en œuvre des composés de départ portant les substituants correspondants.

21. Procédé selon la revendication 20, pour la préparation de la
rac-(9a bêtaH)-7 bêta-(o-chlorophényl)-octahydro-2H-quinolizine-2 alpha-yl)-p-fluorophénylcétone,
à l'état du racémate ou des énantiomères, ainsi que des sels de ces composés formés par addition avec des acides, caractérisé en ce que l'on met en œuvre des composés de départ portant les substituants correspondants.

22. Procédé selon la revendication 1, pour la préparation de composés de formule générale

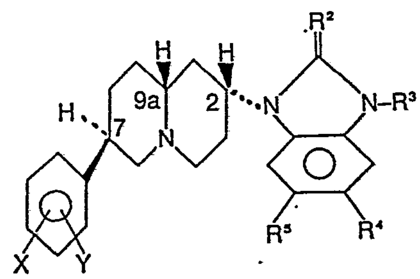

IV–1

dans laquelle X, Y et $R^2$ à $R^5$ ont les significations indiquées dans la revendication 1, à l'état du racémate ou des énantiomères, ainsi que des sels de ces composés formés par addition avec acides, caractérisé en ce que l'on met en œuvre des composés de départ portant les substituants correspondants.

23. Procédé selon la revendication 22, pour la préparation de la
rac-1-[(9a bêtaH)-7 bêta-(o-fluorophényl)-octahydro-2H-quinolizine-2 alpha-yl]-5-bromo-2-benzimidazolinone,
à l'état du racémate ou des énantiomères, ainsi que des sels de ces composés formés par addition avec des acides, caractérisé en ce que l'on met en œuvre des composés de départ portant les substituants correspondants.

24. Procédé selon la revendication 22, pour la préparation de la
rac-1-[(9a bêtaH)-7 bêta-(o-fluorophényl)-octahydro-2H-quinolizine-2 alpha-yl]-5-iodo-2-benzimidazolinone,
à l'état du racémate ou des énantiomères, ainsi que des sels de ces composés formés par addition avec des acides, caractérisé en ce que l'on met en œuvre des composés de départ portant les substituants correspondants.

25. Procédé selon la revendication 22, pour la préparation de la

rac-1-[(9a bêtaH)-7 bêta-(o-fluorophényl)-octahydro-2H-quinolizine-2 alpha-yl]-5-cyano-2-benzimidazolinone,

à l'état du racémate ou des énantiomères, ainsi que des sels de ces composés formés par addition avec des acides, caractérisé en ce que l'on met en œuvre des composés de départ portant les substituants correspondants.

26. Procédé selon la revendication 1, pour la préparation de composés de formule générale

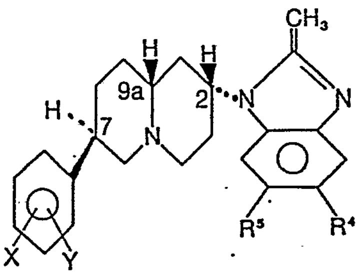

IV-2

dans laquelle X, Y, $R^4$ et $R^5$ ont les significations indiquées dans la revendication 1, à l'état du racémate ou des énantiomères, ainsi que des sels de ces composés formés par addition avec des acides, caractérisé en ce que l'on met en œuvre des composés de départ portant les substituants correspondants.